(19) Europäisches Patentamt
European Patent Office
Office européen des brevets

(11) EP 4 389 891 A1

(12) **EUROPEAN PATENT APPLICATION**
published in accordance with Art. 153(4) EPC

(43) Date of publication:
**26.06.2024 Bulletin 2024/26**

(21) Application number: **22857087.5**

(22) Date of filing: **19.08.2022**

(51) International Patent Classification (IPC):
***C12N 15/113*** (2010.01) ***A61K 31/713*** (2006.01)
***A61K 48/00*** (2006.01) ***A61P 21/00*** (2006.01)

(52) Cooperative Patent Classification (CPC):
**A61K 31/713; A61K 48/00; A61P 21/00; C12N 15/113**

(86) International application number:
**PCT/JP2022/031385**

(87) International publication number:
**WO 2023/022229 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.08.2021 JP 2021134443**

(71) Applicant: **National University Corporation Tokyo Medical and Dental University**
**Tokyo 113-8510 (JP)**

(72) Inventors:
- **YOKOTA Takanori**
  **Tokyo 113-8510 (JP)**
- **NAGATA Tetsuya**
  **Tokyo 113-8510 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **MODIFIED HETERONUCLEIC ACID CONTAINING MORPHOLINO NUCLEIC ACID**

(57) An object of the present invention is to provide a novel double-stranded nucleic acid complex having an excellent activity. Provided is a double-stranded nucleic acid complex comprising a first nucleic acid strand and a second nucleic acid strand, wherein: said first nucleic acid strand comprises an artificial nucleic acid, and is capable of hybridizing to at least part of a target gene or a transcription product thereof, and can induce exon skipping, exon inclusion and/or steric blocking to said target gene or transcription product thereof, said second nucleic acid strand comprises a base sequence complementary to at least part of said first nucleic acid strand, and comprises at least one blocking region which is resistant to a nuclease which is present in a body fluid, and at least one cleavage region which undergoes degradation by a nuclease which is present intracellularly, and said cleavage region comprises two or more consecutive sugar-unmodified nucleosides.

Fig. 3

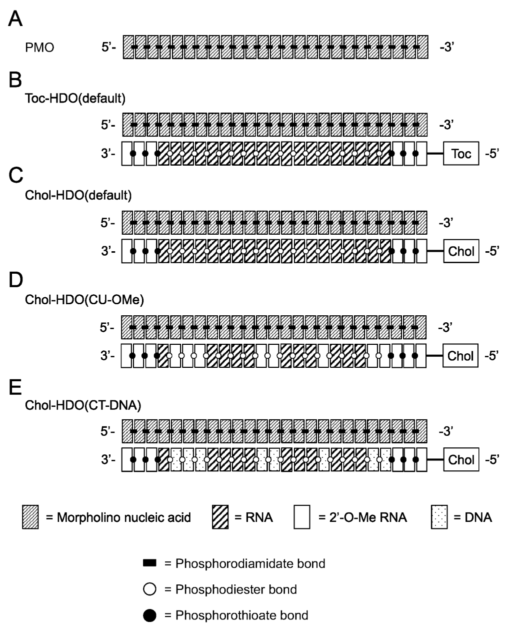


EP 4 389 891 A1

## Description

### Technical Field

The present invention relates to a double-stranded nucleic acid complex comprising a morpholino nucleic acid, and a pharmaceutical composition or the like comprising the same.

### Background Art

In recent years, an oligonucleotide has been drawing attention in the ongoing development of a pharmaceutical called a nucleic acid medicine, and in particular, development of a nucleic acid medicine utilizing the antisense method is actively carried out from the viewpoint of high selectivity on the target gene and low toxicity. The antisense method is a method comprising selectively modifying or inhibiting the expression of a protein encoded by a target gene or the activity of miRNA by introducing into a cell an oligonucleotide complementary to a target sense strand that is a partial sequence of mRNA or miRNA transcribed from the target gene (antisense oligonucleotide, herein often referred to as "ASO").

As a nucleic acid utilizing the antisense method, the present inventors have developed a double-stranded nucleic acid complex (heteroduplex oligonucleotide, HDO) in which an antisense oligonucleotide and a complementary strand thereto are annealed (Patent Literature 1, Non-Patent Literature 1 and 2).

The main mechanism of action of the double-stranded nucleic acid complex has conventionally been considered as follows. That is, when the double-stranded nucleic acid complex is introduced into a cell, an RNA region in the complementary strand is cleaved by RNase H, releasing the antisense oligonucleotide. This antisense oligonucleotide can, for example, function to modify the activity or function of a transcription product (see, for example, Patent Literature 2).

On the other hand, a function of a double-stranded nucleic acid complex that is not dependent on the above-described mechanism of action has also been revealed. For example, when the antisense oligonucleotide in a double-stranded nucleic acid complex is composed only of morpholino nucleic acids, it is considered that the double-stranded nucleic acid complex does not have an RNase H-dependent activity. However, it has been revealed that, even in such a case, the complex can provide an antisense effect (Patent Literature 3). As an example of an antisense nucleic acid medicine consisting of morpholino nucleic acids, viltolarsen (VILTEPSO) has been developed for Duchenne muscular dystrophy. It is possible that the efficacy of the morpholino nucleic acid can be enhanced by applying the above-described technology of forming a heteronucleic acid.

### Citation List

#### Patent Literature

Patent Literature 1: WO2013/089283
Patent Literature 2: WO2014/192310
Patent Literature 3: PCT/JP2021/010214

#### Non-Patent Literature

Non-Patent Literature 1: Nishina K, et. al., "DNA/RNA heteroduplex oligonucleotide for highly efficient gene silencing", Nature Communication, 2015, 6:7969.

Non-Patent Literature 2: Asami Y, et al., "Drug delivery system of therapeutic oligonucleotides", Drug Discoveries & Therapeutics. 2016; 10(5):256-262.

### Summary of Invention

### Technical Problem

In order that a double-stranded nucleic acid complex can function *in vivo,* it is generally desirable that degradation (*e.g.*, degradation by a nucleic acid-degrading enzyme such as RNase or DNase) and excretion (*e.g.*, renal excretion or the like) are avoided in the blood and the spinal fluid. In addition, in order that a double-stranded nucleic acid complex

can function on a target nucleic acid after transitioning into the target cell, it is considered to be necessary that an antisense oligonucleotide is released from the double-stranded nucleic acid complex in the cell.

However, in the case of a double-stranded nucleic acid complex having an antisense oligonucleotide composed of *e.g.*, morpholino nucleic acids, neither a method of enhancing the efficacy of the complex nor a desirable nucleic acid structure to be used as a complementary strand is known.

An object of the present disclosure is to provide a novel double-stranded nucleic acid complex having an excellent activity.

Solution to Problem

The present inventors have carried out studies intensively to further enhance the efficacy of a double-stranded nucleic acid complex having an antisense oligonucleotide composed of morpholino nucleic acids, and have introduced a deoxyribonucleoside or a 2'-modified nucleoside into a nucleoside comprising a pyrimidine base in the complementary strand of the morpholino nucleic acid. As a result, the present inventors have found that the stability of the double-stranded nucleic acid complex is increased, and the antisense effect through systemic administration is greatly increased. The antisense effect obtained by this method is surprisingly 5 to 10 times as high as the effect of a control double-stranded nucleic acid complex, and dozens of times as high as the effect of a single-stranded morpholino nucleic acid. The present invention is based on the above-described findings, and provides the following.

(1) A double-stranded nucleic acid complex comprising a first nucleic acid strand and a second nucleic acid strand, wherein: said first nucleic acid strand comprises an artificial nucleic acid, and is capable of hybridizing to at least part of a target gene or a transcription product thereof, and can induce exon skipping, exon inclusion and/or steric blocking to said target gene or transcription product thereof, said second nucleic acid strand comprises a base sequence complementary to at least part of said first nucleic acid strand, and comprises at least one blocking region which is resistant to a nuclease which is present in a body fluid, and at least one cleavage region which undergoes degradation by a nuclease which is present intracellularly, and said cleavage region comprises two or more consecutive sugar-unmodified nucleosides.

(2) The double-stranded nucleic acid complex of (1), wherein said artificial nucleic acid comprises any one or more of a morpholino nucleic acid, a 2'-O-methyl-modified nucleoside, a 2'-O-methoxyethyl-modified nucleoside, a tricyclo-DNA, a peptide nucleic acid, or a 2'-O-[2-(N-methylcarbamoyl)ethyl]-modified nucleoside.

(3) The double-stranded nucleic acid complex of (1) or (2), wherein said blocking region comprises at least one or more selected from the group consisting of a deoxyribonucleoside, a 2'-modified nucleoside, a 5'-modified nucleoside, and a bridged nucleoside which comprises a pyrimidine base.

(4) The double-stranded nucleic acid complex of (3), wherein said 2'-modified nucleoside is selected from the group consisting of a 2'-O-methyl-modified nucleoside, a 2'-O-methoxyethyl-modified nucleoside, and a 2'-O-[2-(N-methylcarbamoyl)ethyl]-modified nucleoside.

(5) The double-stranded nucleic acid complex of (3) or (4), wherein said 5'-modified nucleoside is a 5'-cp-modified nucleoside, a 5'-methyl-modified nucleoside, or a 5'-dimethyl-modified nucleoside.

(6) The double-stranded nucleic acid complex of any one of (3) to (5), wherein said bridged nucleoside is selected from the group consisting of an LNA nucleoside, a 2',4'-BNA$^{NC}$ nucleoside, a cEt BNA nucleoside, an ENA nucleoside, an AmNA nucleoside, a GuNA nucleoside, an scpBNA nucleoside, an scpBNA2 nucleoside, and a BANA3 nucleoside.

(7) The double-stranded nucleic acid complex of any one of (1) to (6), wherein said second nucleic acid strand does not comprise two or more consecutive natural ribonucleosides comprising a pyrimidine base.

(8) The double-stranded nucleic acid complex of any one of (1) to (7), wherein all of the nucleosides that comprise a pyrimidine base in said second nucleic acid strand are deoxyribonucleosides, 2'-modified nucleosides, 5'-modified nucleosides, bridged nucleosides, or a combination thereof.

(9) The double-stranded nucleic acid complex of any one of (1) to (8), wherein said cleavage region comprises one to ten consecutive sugar-unmodified nucleosides.

(10) The double-stranded nucleic acid complex of any one of (1) to (9), wherein the nucleic acids in said first nucleic acid strand consist of morpholino nucleic acids.

(11) The double-stranded nucleic acid complex of any one of (1) to (10), wherein said second nucleic acid strand comprises a non-complementary base, and/or one or more insertion sequences and/or deletions, relative to said first nucleic acid strand.

(12) The double-stranded nucleic acid complex of (11), wherein said second nucleic acid strand comprises one to three said non-complementary bases.

(13) The double-stranded nucleic acid complex of (11) or (12), wherein said insertion sequence consists of one to

eight bases.

(14) The double-stranded nucleic acid complex of any one of (11) to (13), wherein said deletion consists of consecutive one to four bases.

(15) The double-stranded nucleic acid complex of any one of (1) to (14), wherein: in said sugar-unmodified nucleosides, the internucleoside linkage between a deoxyribonucleoside and a deoxyribonucleoside is a phosphodiester bond, and the internucleoside linkage between a deoxyribonucleoside and a ribonucleoside or the internucleoside linkage between a ribonucleoside and a ribonucleoside is a phosphodiester linkage and/or a phosphorothioate bond.

(16) The double-stranded nucleic acid complex of any one of (1) to (15), wherein said second nucleic acid strand is at least 8 bases in length.

(17) The double-stranded nucleic acid complex of any one of (1) to (16), wherein the base length of said second nucleic acid strand is shorter than the base length of the first nucleic acid strand.

(18) The double-stranded nucleic acid complex of any one of (1) to (17), wherein said second nucleic acid strand comprises at least one overhang region at one or both of the 5' end side and 3' end side thereof.

(19) The double-stranded nucleic acid complex of (18), wherein said overhang region is one to 20 bases in length.

(20) The double-stranded nucleic acid complex of (18) or (19), wherein said overhang region comprises at least one deoxyribonucleoside and/or non-natural nucleoside.

(21) The double-stranded nucleic acid complex of any one of (1) to (20), wherein said second nucleic acid strand comprises at least one non-natural nucleoside at the 5' end and/or 3' end thereof.

(22) The double-stranded nucleic acid complex of any one of (1) to (21), wherein said second nucleic acid strand is bound to a ligand.

(23) The double-stranded nucleic acid complex of (22), wherein said ligand is any one or more selected from the group consisting of a small molecule, a peptide, a lipid, and a nucleic acid aptamer.

(24) The double-stranded nucleic acid complex of (23), wherein said peptide is an antibody or an active fragment thereof.

(25) The double-stranded nucleic acid complex of (23), wherein said lipid is cholesterol or an analog thereof, or tocopherol or an analog thereof.

(26) The double-stranded nucleic acid complex of any one of (22) to (25), wherein said ligand is bound to the 5' end and/or 3' end of said second nucleic acid strand.

(27) The double-stranded nucleic acid complex of any one of (1) to (26), wherein all or a part of the internucleoside linkages in said first nucleic acid strand and/or said second nucleic acid strand is a modified internucleoside linkage.

(28) The double-stranded nucleic acid complex of (27), wherein said modified internucleoside linkage is a phosphorothioate linkage and/or a boranophosphate linkage.

(29) The double-stranded nucleic acid complex of any one of (1) to (28), wherein said first nucleic acid strand and said second nucleic acid strand are bound via a linker.

(30) The double-stranded nucleic acid complex of (29), wherein said linker consists of nucleic acids.

(31) The double-stranded nucleic acid complex of any one of (1) to (30), wherein said body fluid is blood, spinal fluid, or lymph fluid.

(32) A pharmaceutical composition comprising the double-stranded nucleic acid complex of any one of (1) to (31) as an active ingredient.

(33) The pharmaceutical composition of (32) for increasing RNA expression or reducing RNA expression by exon skipping, exon inclusion and/or steric blocking, or regulating splice variants.

(34) The pharmaceutical composition of (32) or (33) for treating skeletal muscle dysfunction or cardiac dysfunction.

(35) The pharmaceutical composition of (34), wherein said skeletal muscle dysfunction or cardiac dysfunction is muscular dystrophy.

(36) The pharmaceutical composition of (35), wherein said muscular dystrophy is myotonic dystrophy or Duchenne muscular dystrophy.

(37) The pharmaceutical composition of (32) or (33) for treating brain disorder, spinal cord disorder, or peripheral nerve disorder.

(38) The pharmaceutical composition of any one of (32) to (37), wherein the pharmaceutical composition is administered by intravenous administration, intramuscular administration, intraocular administration, transnasal administration, oral administration, inhalation administration, or subcutaneous administration.

(39) The pharmaceutical composition of (38), wherein 0.1 mg/kg to 100 mg/kg of said double-stranded nucleic acid complex is administered.

(40) The pharmaceutical composition of any one of (32) to (37), wherein the pharmaceutical composition is administered by intraventricular administration or intrathecal administration.

(41) The pharmaceutical composition of (40), wherein said intrathecal administration is lumbar puncture.

(42) The pharmaceutical composition of (40) or (41), wherein said double-stranded nucleic acid complex is administered at 0.1 mg to 200 mg.

(43) The pharmaceutical composition of any one of (32) to (42), wherein the stability of said double-stranded nucleic acid complex in the blood and/or spinal fluid is improved.

The contents of the disclosures in Japanese Patent Application No. 2021-134443 which forms the basis for priority of the present application are incorporated herein.

Advantageous Effects of Invention

The present invention provides a novel double-stranded nucleic acid complex having an excellent activity.

Brief Description of Drawings

[Figure 1] Figure 1 shows the structures of various bridged nucleic acids.
[Figure 2] Figure 2 shows the structures of various natural nucleotides or non-natural nucleotides.
[Figure 3] Figure 3 schematically shows the structures of nucleic acids used in Examples 1 and 2. Toc-HDO (default) represents a conventional Toc-HDO; Chol-HDO (default) represents a conventional Chol-HDO; and Chol-HDO (CU-OMe) and Chol-HDO (CT-DNA) each represents a Chol-HDO of the present invention.
[Figure 4] Figure 4 shows the exon skipping effect on the dystrophin mRNA in the heart muscle of mice to which various nucleic acid agents were administered by intravenous injection. The error bars indicate standard errors.
[Figure 5] Figure 5 shows the exon skipping effect on the dystrophin mRNA in the diaphragm mice to which various nucleic acid agents were administered by intravenous injection. The error bars indicate standard errors.
[Figure 6] Figure 6 shows the exon skipping effect on the dystrophin mRNA in the paraspinal muscle of mice to which various nucleic acid agents were administered by intravenous injection. The error bars indicate standard errors.
[Figure 7] Figure 7 shows the exon skipping effect on the dystrophin mRNA in the quadriceps of mice to which various nucleic acid agents were administered by intravenous injection. The error bars indicate standard errors.
[Figure 8] Figure 8 shows the exon skipping effect on the dystrophin mRNA in the tibialis anterior of mice to which various nucleic acid agents were administered by intravenous injection. The error bars indicate standard errors.
[Figure 9] Figure 9 shows the exon skipping effect on the dystrophin mRNA in the triceps of mice to which various nucleic acid agents were administered by intravenous injection. The error bars indicate standard errors.
[Figure 10] Figure 10 shows the results of electrophoresis for examining the stability of HDO (PMO/cRNA) and HDO (PMO/cDNA) in the human spinal fluid after mixing with the human spinal fluid and incubating for certain periods of time (0 minutes, 10 minutes, 6 hours, 24 hours, and 5 days). Figure 10A shows the results of HDO (PMO/cRNA). The dotted line indicates band shift toward the lower mobility direction. With HDO (PMO/cRNA), it is shown that the complementary strand (cRNA) was degraded in the human spinal fluid, generating a single-stranded PMO molecule. Figure 10B shows the results of HDO (PMO/cDNA). The arrow indicates the band of double-stranded nucleic acid complex.
[Figure 11] Figure 11 shows the results of electrophoresis for examining the stability of Chol-HDO (default), Chol-HDO (CU-OMe), and Chol-HDO (CT-DNA) in the human spinal fluid after mixing with the human spinal fluid, and incubating for certain periods of time (0 minutes, 6 hours, and 24 hours). Figure 11A shows the results of Chol-HDO (default). Figure 11B shows the results of Chol-HDO (CU-OMe). Figure 11C shows the results of Chol-HDO (CT-DNA). The arrow indicates the band of double-stranded nucleic acid complex.

Description of Embodiments

1. Double-stranded nucleic acid complex

1-1. Overview

A first aspect of the present invention is a double-stranded nucleic acid complex. The double-stranded nucleic acid complex of the present invention comprises a first nucleic acid strand and a second nucleic acid strand. The first nucleic acid strand comprises an artificial nucleic acid, and is capable of hybridizing to at least part of a target gene or a transcription product thereof, and can induce exon skipping, exon inclusion and/or steric blocking to said target gene or transcription product thereof. The second nucleic acid strand comprises a base sequence complementary to at least part of the first nucleic acid strand, and comprises at least one blocking region which is resistant to a nuclease which is present in a body fluid, and at least one cleavage region which undergoes degradation by a nuclease which is present intracellularly.

In the double-stranded nucleic acid complex of the present invention, the degradation of the second nucleic acid strand can be reduced based on the blocking region in the blood and the spinal fluid, whereas the cleavage region can promote the cleavage of the second nucleic acid strand after the complex transitions into cells. A double-stranded nucleic acid complex of the present invention can suppress or increase the expression amount of a transcription product or translation product of a target gene in various tissues and organs of a subject, inhibit the function of a transcription product or translation product of a target gene, or induce steric blocking, splicing switching, RNA editing, exon skipping, or exon inclusion, based on the antisense effect. The *in vivo* stability of a double-stranded nucleic acid complex of the present invention is high, and the antisense effect such as exon skipping is high.

1-2. Definition of terms

A "transcription product" of a target gene means herein any RNA that is synthesized by an RNA polymerase and is a direct target of the nucleic acid complex of the present invention. Specifically, mRNA transcribed from a target gene (comprising, *e.g.*, mature mRNA, mRNA precursor, and mRNA without base modification), non-coding RNA (ncRNA) such as miRNA, long non-coding RNA (lncRNA), and natural antisense RNA can be included.

A "target gene" means herein a gene wherein the expression level of transcription product or translation product thereof can be reduced or increased; a gene wherein the function of transcription product or translation product thereof can be inhibited; or a gene for which steric blocking, splicing switching, RNA editing, exon skipping, or exon inclusion can be induced. There is no particular restriction on the kind of a target gene, as long as it is expressed *in vivo.* Examples thereof include a gene which is derived from an organism into which a double-stranded nucleic acid complex of the present invention is to be introduced, such as a gene whose expression is increased in various diseases. Specific examples thereof include a scavenger receptor B1 (often referred to herein as "SR-B1") gene, and a metastasis associated lung adenocarcinoma transcript 1 (herein often referred to as "Malat1") gene, a DMPK (dystrophia myotonica-protein kinase) gene, and a dystrophin gene.

A "target transcription product" means herein any RNA that is synthesized by an RNA polymerase and is a direct target of the nucleic acid complex of the present invention. In general, it is a "transcription product of a target gene". Specifically, mRNA transcribed from a target gene (comprising, *e.g.*, mature mRNA, mRNA precursor, and mRNA without base modification), non-coding RNA (ncRNA) such as miRNA, long non-coding RNA (lncRNA), and natural antisense RNA can be included. Examples of a transcription product of a target gene may comprise SR-B1 mRNA which is a transcription product of the SR-B1 gene, Malat1 non-coding RNA which is a transcription product of the Malat1 gene, DMPK mRNA which is a transcription product of a DMPK gene, and dystrophin mRNA which is a transcription product of the dystrophin gene or a precursor thereof (pre-mRNA).

Specific examples of a target transcription product include the exon 23/intron 23 boundary region of Dystrophin pre-mRNA (GenBank accession number: NC_000086.7), for example, positions 83803482-83803566, *e.g.*, 83803512-83803536. As other specific examples of a target transcription product, the base sequence of a murine DMPK mRNA is shown in SEQ ID NO: 7, and the base sequence of a human DMPK mRNA is shown in SEQ ID NO: 8. In this regard, in all of SEQ ID NOs: 7 to 8, the base sequences of mRNA are replaced with the base sequences of DNA. The base sequence information for these genes and transcription products can be obtained from publicly known databases, such as the database of NCBI (The U.S. National Center for Biotechnology Information).

The base sequence of a publicly known antisense medicine may be also utilized. For example, the base sequence shown in SEQ ID NO: 9 constituting ISIS 598769 (IONIS) which is a therapeutic drug for myotonic dystrophy and related to its causative gene, namely DMPK gene; the base sequence shown in SEQ ID NO: 10 constituting Eteplirsen (Exondys 51, Sarepta Therapeutics), which is known as a therapeutic drug for Duchenne muscular dystrophy and induces exon skipping in pre-mRNA of the dystrophin gene; the base sequence shown in SEQ ID NO: 11 constituting Golodirsen (Sarepta Therapeutics); the base sequence shown in SEQ ID NO: 12 constituting NS-065/NCNP-01 (Nippon Shinyaku Co., Ltd.); the base sequence shown in SEQ ID NO: 13 constituting Casimersen (Sarepta Therapeutics); the base sequence shown in SEQ ID NO: 14 constituting Renadirsen; the base sequence shown in SEQ ID NO: 15 constituting Suvodirsen; and the base sequence shown in SEQ ID NO: 16 constituting Suvodirsen may be used.

An "antisense oligonucleotide (ASO)" or "antisense nucleic acid" herein refers to a single-stranded oligonucleotide that comprises a base sequence capable of hybridizing (that is, complementary) to at least a part of a target transcription product (mainly, a transcription product of a target gene), and can produce an antisense effect on the target transcription product. In the double-stranded nucleic acid complex of the present invention, the first nucleic acid strand functions as ASO, and its target region may comprise 3'UTR, 5'UTR, exon, intron, coding region, translation initiation region, translation termination region, or any other nucleic acid region. The target region of a target transcription product may be at least 8 bases in length, for example, 10 to 35 bases in length, 12 to 25 bases in length, 13 to 20 bases in length, 14 to 19 bases in length, 15 to 18 bases in length, 13 to 22 bases in length, 16 to 22 bases in length, or 16 to 20 bases in length.

An "antisense effect" means an effect of regulating expression or editing of a target transcription product by

hybridization of ASO to the target transcription product (*e.g.* RNA sense strand). The phrase "regulating expression or editing of a target transcription product" includes suppression or reduction of the expression of a target gene or the expression amount of a target transcription product ("expression amount of a target transcription product" is herein often referred to as "expression level of a target transcription product"), inhibition of translation, RNA editing, a splicing function modifying effect (*e.g.,* splicing switching, exon inclusion, and exon skipping), or degradation of a transcription product. For example, in the case of post-transcriptional inhibition of a target gene, when an RNA oligonucleotide is introduced into a cell as ASO, the ASO forms a partial double strand by annealing to mRNA which is a transcription product of a target gene. This partial double strand serves as a cover to prevent translation by ribosomes, so as to inhibit the expression of the target protein encoded by the target gene at the translation level (steric blocking). On the other hand, when an oligonucleotide comprising DNA is introduced into a cell as ASO, a partial DNA-RNA heteroduplex is formed. This hetero double-stranded structure is recognized by RNase H, and as a result mRNA of the target gene is degraded and the expression of the protein encoded by the target gene is inhibited at the expression level. In addition, an antisense effect can also be produced for an intron in an mRNA precursor as a target. Further, an antisense effect can also be produced for miRNA as a target. In this case, as a result of functional inhibition of the miRNA, the expression of the gene whose expression is normally regulated by the miRNA may be increased. In one embodiment, expression regulation of a target transcription product may be a decrease in the amount of a target transcription product.

The antisense effect can be measured, for example, as follows: a subject nucleic acid compound is administered to a subject (for example, a mouse); and, for example, after several days (for example, after 2 to 7 days), a measurement is made of the expression amount of a target gene or the level (amount) of the target transcription product (for example, the amount of mRNA, the amount of RNA such as microRNA, the amount of cDNA, the amount of protein, or the like), wherein the expression of the target gene is regulated by the antisense effect provided by the subject nucleic acid compound. For example, when the expression amount measured of the target gene or the level measured of the target transcription product is decreased by at least 10%, at least 20%, at least 25%, at least 30%, or at least 40%, compared with a negative control (for example, vehicle administration), the measurement shows that the subject nucleic acid compound can produce an antisense effect (for example, a decrease in the amount of the target transcription product).

The number, kind, and position of a non-natural nucleotide in a nucleic acid strand may influence the antisense effect and the like provided by the nucleic acid complex. The choice of a modification may vary depending on the sequence of a target gene or the like, but those skilled in the art can determine a suitable embodiment by referring to the descriptions in the literature related to the antisense method (*e.g.,* WO 2007/143315, WO 2008/043753, and WO 2008/049085). Furthermore, when the antisense effect of a nucleic acid complex after the modification is measured and the obtained measured value is not significantly lower than the measured value of the nucleic acid complex before the modification (*e.g.*, when the measured value obtained after the modification is 70% or more, 80% or more, or 90% or more of the measured value of the nucleic acid complex before the modification), a relevant modification may be evaluated.

A "translation product of the target gene" means herein any polypeptide or protein that is synthesized by translation of a target transcription product or a transcription product of a target gene which is a direct target of a nucleic acid complex of the present invention. Examples of a translation product of the target gene comprise a DMPK protein, which is a translation product of the DMPK gene, a dystrophin protein, which is a translation product of the dystrophin gene, and the like. For example, inducing exon skipping and/or exon inclusion in a target gene can increase the expression amount of a desired translation product.

Herein, an "aptamer" refers to a nucleic acid molecule that specifically binds to a particular target molecule which is intracellular, on the cell membrane, or extracellular, such as a target molecule on the cell membrane or an extracellular target molecule. An aptamer can be produced by a method known in the art, for example, an *in vitro* sorting method using the SELEX (systematic evolution of ligands by exponential enrichment) method.

Herein, "decoy" refers to a nucleic acid having the sequence of a binding site of transcription factor (*e.g.*, NF-kB) or a similar sequence, which is introduced into a cell as a "decoy" to inhibit the function of a transcription factor (inhibit transcription in the case of a transcription activator or promote transcription in the case of a transcription repressor). A decoy nucleic acid can be easily designed based on information about a binding sequence of a target transcription factor.

Herein, "bait" refers to a nucleic acid molecule that specifically binds to a particular target molecule in a cell and modifies a function of the target molecule. A target that interacts with a bait is also referred to as a "prey".

The term "nucleic acid" or "nucleic acid molecule" used herein may refer to a nucleotide or nucleoside of a monomer, and may mean an oligonucleotide consisting of a plurality of monomers, or means a polynucleotide in the case of a polymer. A "natural nucleic acid" refers to a naturally-occurring nucleic acid. Examples of the natural nucleic acid include a natural nucleoside, natural nucleotide, and the like, as described below. A "non-natural nucleic acid" or "artificial nucleic acid" refers to any nucleic acid other than a natural nucleic acid. Examples of the non-natural nucleic acid or the artificial nucleic acid include a non-natural nucleoside, non-natural nucleotide, and the like, as described below.

A "nucleic acid strand" or simply "strand" herein means an oligonucleotide or a polynucleotide. A full length strand or a partial length strand of a nucleic acid strand can be produced by a chemical synthesis using an automated synthesizer, or by an enzymatic step using a polymerase, a ligase, or a restricted reaction. A nucleic acid strand may

comprise a natural nucleotide and/or a non-natural nucleotide.

A "nucleoside" generally means a molecule consisting of a combination of a base and a sugar. The sugar moiety of a nucleoside is usually, but not limited to, composed of pentofuranosyl sugar, and specific examples thereof include ribose and deoxyribose. The base moiety of nucleoside (nucleobase) is usually a heterocyclic base moiety. Without limitation, examples thereof include adenine, cytosine, guanine, thymine, or uracil as well as other modified nucleobases (modified bases).

A "nucleotide" refers to a molecule in which a phosphate group is covalently bound to the sugar moiety of the nucleoside. In the case of a nucleotide comprising pentofuranosyl sugar, a phosphate group is usually linked to a hydroxyl group at the 2', 3', or 5' position of the sugar.

An "oligonucleotide" refers to a linear oligomer formed by linking several to dozens of neighboring nucleotides through a covalent bond between a hydroxyl group and a phosphate group in the sugar moiety. Further, a "polynucleotide" refers to a linear polymer formed by linking with covalent bonds dozens or more, preferably hundreds or more of nucleotides, namely more nucleotides than in an oligonucleotide. It is considered that the phosphate group generally forms an internucleoside linkage inside the structure of an oligonucleotide or a polynucleotide.

A "natural nucleoside" refers herein to a nucleoside that exists in nature. Examples thereof include a ribonucleoside consisting of a ribose and the aforementioned base such as adenine, cytosine, guanine, or uracil, or a deoxyribonucleoside consisting of a deoxyribose and the aforementioned base such as adenine, cytosine, guanine, or thymine. In this regard, a ribonucleoside found in RNA, and a deoxyribonucleoside found in DNA are herein often referred to as "DNA nucleoside" and "RNA nucleoside", respectively.

A "natural nucleotide" means herein a nucleotide that exists in nature, namely a molecule in which a phosphate group is covalently bound to the sugar moiety of the aforementioned natural nucleoside. Examples thereof include a ribonucleotide which is known as a constituent of RNA, and in which a phosphate group is bound to a ribonucleoside, and a deoxyribonucleotide, which is known as a constituent of DNA, and in which a phosphate group is bound to a deoxyribonucleoside.

A "non-natural nucleotide" means herein any nucleotide other than a natural nucleotide. For example, it comprises a modified nucleotide and a nucleotide mimic. A "modified nucleotide" means herein a nucleotide having any one or more of a modified sugar moiety, a modified internucleoside linkage, and a modified nucleobase. The term "nucleotide mimic" herein comprises a structure used to substitute a nucleoside and a bond (linkage) at one or more positions in an oligomer compound. Examples of the nucleotide mimic comprise a peptide nucleic acid, and a morpholino nucleic acid (morpholino linked with -N(H)-C(=O)-O- or other non-phosphodiester linkages). The peptide nucleic acid (PNA) is a nucleotide mimic having a main chain in which N-(2-aminoethyl)glycine in place of a sugar is linked with an amide linkage. A nucleic acid strand comprising a non-natural oligonucleotide herein has in many cases preferable properties, such as enhanced cellular uptake, enhanced affinity for a target nucleic acid, increased stability in the presence of a nuclease, and increase in inhibitory activity. Therefore, it is more preferable than a natural nucleotide.

A "non-natural nucleoside" means herein any nucleoside other than a natural nucleoside. For example, it comprises a modified nucleoside and a nucleoside mimic. A "modified nucleoside" means herein a nucleoside having a modified sugar moiety and/or a modified nucleobase.

A "mimic" refers herein to a functional group that substitutes a sugar, a nucleobase, and/or an internucleoside linkage. In general, a mimic is used in place of a sugar or a combination of a sugar-internucleoside linkage, and a nucleobase is maintained for hybridization to a target to be selected. The term "nucleoside mimic" used herein comprises a structure to be used for substituting a sugar at one or more positions of an oligomer compound, or substituting a sugar and a base, or substituting a bond between monomer subunits constituting an oligomer compound. An "oligomer compound" means a polymer composed of linked monomer subunits that can at least hybridize to a region of a nucleic acid molecule. Examples of a nucleoside mimic comprise a morpholino, cyclohexenyl, cyclohexyl, tetrahydropyranyl, bicyclic or tricyclic sugar mimic, such as a nucleoside mimic having a non-furanose sugar unit.

A "modified sugar" refers to a sugar in which a natural sugar moiety (*i.e.*, sugar moiety found in DNA(2'-H) or RNA(2'-OH)) has undergone a substitution and/or any change. "Sugar modification" refers to substitution and/or any change from a natural sugar moiety. A nucleic acid strand may comprise in some cases one or more modified nucleosides comprising a modified sugar. A "sugar-modified nucleoside" means a nucleoside having a modified sugar moiety. Such a sugar-modified nucleoside can confer a beneficial biological property such as enhanced nuclease stability, increased binding affinity, or the like to a nucleic acid strand. In a specific embodiment, a nucleoside comprises a chemically modified ribofuranose ring moiety. Examples of a chemically modified ribofuranose ring comprise, but are not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a $C_1$-$C_{12}$ alkyl, or a protecting group), and a combination thereof.

Examples of the sugar-modified nucleoside comprise, but are not limited to, a nucleoside comprising a substituent such as 5'-vinyl, 5'-methyl (R or S), 5'-allyl (R or S), 4'-S, 2'-F(2'-fluorogroup), 2'-$OCH_3$ (2'-OMe group or 2'-O-methyl group), 2'-O-[2-(N-methylcarbamoyl)ethyl] (2'-O-MCE group), and 2'-O-methoxyethyl (2'-O-MOE or 2-$O(CH_2)_2OCH_3$). A substituent at the 2' position may be selected from allyl, amino, azide, thio, -O-allyl, -O-$C_1$-$C_{10}$ alkyl, -$OCF_3$, -$O(CH_2)_2SCH_3$, -$O(CH_2)_2$-O-N(Rm)(Rn), and O-$CH_2$-C(=O)-N(Rm)(Rn), wherein Rm and Rn are independently H or a substituted or unsubstituted $C_1$-$C_{10}$ alkyl. A "2'-modified sugar" means a furanosyl sugar modified at the 2' position. A nucleoside comprising a 2'-modified sugar may be referred to as a "2'-sugar-modified nucleoside" or a "2'-modified nucleoside". In addition, a "5'-modified sugar" means a furanosyl sugar modified at the 5' position. A nucleoside comprising a 5'-modified sugar may be referred to as a "5'-sugar-modified nucleoside" or a "5'-modified nucleoside".

A "bicyclic nucleoside" refers to a modified nucleoside comprising a bicyclic sugar moiety. A nucleic acid comprising a bicyclic sugar moiety is commonly referred to as bridged nucleic acid (BNA). A nucleoside comprising a bicyclic sugar moiety is sometimes referred to as a "bridged nucleoside (bridged-type nucleoside)", "bridged-type non-natural nucleoside", or "BNA nucleoside". Some examples of a bridged nucleic acid are shown in Figure 1.

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) or of BNA nucleosides may be described as having a carbon atom at the 2' position and a carbon atom at the 4' position which are bridged with 4'-$(CH_2)_p$-O-2', 4'-$(CH_2)_p$-$CH_2$-2', 4'-$(CH_2)_p$-S-2', 4'-$(CH_2)_p$-OCO-2', 4'-$(CH_2)_n$-N($R_3$)-O-$(CH_2)_m$-2' [wherein p, m, and n represent integers from 1 to 4, from 0 to 2, and from 1 to 3, respectively; and $R_3$ represents a hydrogen atom, an alkyl group, an alkenyl group, a cycloalkyl group, an aryl group, an aralkyl group, an acyl group, a sulfonyl group, and a unit substituent (*e.g.*, a fluorescently or chemiluminescently labeled molecule, a functional group with nucleic acid cleavage activity, and an intracellular or nuclear localization signal peptide)]. Furthermore, with respect to a BNA or a BNA nucleoside in a certain embodiment, in the $OR_2$ substituent of the carbon atom at the 3' position and the $OR_1$ substituent of the carbon atom at the 5' position, $R_1$ and $R_2$ are typically hydrogen atoms, but may be the same or different from each other, or may also be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or $P(R_4)R_5$ [wherein $R_4$ and $R_5$, may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, a $C_1$-$C_5$ alkoxy group, a $C_1$-$C_5$ alkylthio group, a $C_1$-$C_6$ cyanoalkoxy group, or an amino group substituted with a $C_1$-$C_5$ alkyl group]. Non-limiting examples of such BNA comprise methyleneoxy(4'-$CH_2$-O-2') BNA (LNA, Locked Nucleic Acid®, also known as 2',4'-BNA) (*e.g.*, α-L-methyleneoxy(4'-$CH_2$-O-2') BNA or β-D-methyleneoxy(4'-$CH_2$-O-2') BNA), ethyleneoxy(4'-$(CH_2)_2$-O-2') BNA (also known as ENA), β-D-thio(4'-$CH_2$-S-2') BNA, aminooxy(4'-$CH_2$-O-N($R_3$)-2') BNA, oxyamino(4'-$CH_2$-N($R_3$)-O-2') BNA (also known as 2',4'-$BNA^{NC}$; R=H is 2',4'-$BNA^{NC}$[N-H], R=Me is 2',4'-$BNA^{NC}$[N-Me]), 2',4'-$BNA^{coc}$, 3'-amino-2',4'-BNA, 5'-methyl BNA, (4'-CH($CH_3$)-O-2') BNA (also known as cEt BNA), (4'-CH($CH_2OCH_3$)-O-2') BNA (also known as cMOE BNA), amide BNA (amide-bridged nucleic acid) or (4'-C(O)-N(R)-2') BNA (R=H, or Me) (also known as AmNA; R=H in Figure 1 is AmNA[N-H], R=Me is AmNA[N-Me]), guanidine BNA (also known as GuNA (*e.g.*, R=H is GuNA[N-H], R=Me is GuNA[N-Me]) in Figure 1), amine BNA (also known as 2'-Amino-LNA) (*e.g.*, 3-(Bis(3-aminopropyl)amino)propanoyl substitution product), 2'-O,4'-C-spirocyclopropylene-bridged nucleic acid (also known as scpBNA), and other BNA known to those skilled in the art. Non-limiting examples of such BNA nucleoside comprise methyleneoxy(4'-$CH_2$-O-2') BNA nucleoside (also known as LNA nucleoside or 2',4'-BNA nucleoside) (*e.g.*, α-L-methyleneoxy(4'-$CH_2$-O-2') BNA nucleoside, β-D-methyleneoxy(4'-$CH_2$-O-2') BNA nucleoside), ethyleneoxy(4'-$(CH_2)_2$-O-2') BNA nucleoside (also known as ENA nucleoside), β-D-thio(4'-$CH_2$-S-2') BNA nucleoside, aminooxy(4'-$CH_2$-O-N($R_3$)-2') BNA nucleoside, oxyamino(4'-$CH_2$-N($R_3$)-O-2') BNA nucleoside (also known as 2',4'-$BNA^{NC}$ nucleoside; R=H is 2',4'-$BNA^{NC}$[N-H] nucleoside, and R=Me is 2',4'-$BNA^{NC}$[N-Me] nucleoside), 2',4'-$BNA^{coc}$ nucleoside, 3'-amino-2',4'-BNA nucleoside, 5'-methyl BNA nucleoside, (4'-CH($CH_3$)-O-2') BNA nucleoside (also known as cEt nucleoside), (4'-CH($CH_2OCH_3$)-O-2') BNA nucleoside (also known as cMOE nucleoside), amide BNA nucleoside or (4'-C(O)-N(R)-2') BNA nucleoside (R=H, or Me) (also known as AmNA nucleoside; R=H in Figure 1 is AmNA[N-H] nucleoside, and R=Me is AmNA[N-Me] nucleoside)), guanidine BNA nucleoside (GuNA nucleoside (*e.g.*, R=H in Figure 1 is also known as GuNA[N-H] nucleoside, and R=Me is also known as GuNA[N-Me] nucleoside)), amine BNA nucleoside (also known as 2'-Amino-LNA nucleoside) (*e.g.*, 3-(Bis(3-aminopropyl)amino)propanoyl-substituted nucleoside), 2'-O,4'-C-spirocyclopropylene-bridged nucleoside (also known as scpBNA nucleoside), and other BNA nucleosides known to those skilled in the art.

A "cationic nucleoside" herein is a modified nucleoside existing in cationic form, compared with a neutral form (such as the neutral form of ribonucleoside), at a pH (for example, the physiological pH (approximately 7.4) of a human, a pH of a delivery site (*e.g.*, an organelle, cell, tissue, organ, or organism), or the like). The cationic nucleoside may comprise one or more cationic modified groups at any position of a nucleoside. In one embodiment, the cationic nucleoside is 2'-Amino-LNA nucleoside (e.g., 3-(Bis(3-aminopropyl)amino)propanoyl-substituted nucleoside), aminoalkyl-modified nucleoside (*e.g.*, 2'-O-methyl- and 4'-$CH_2CH_2CH_2NH_2$-substituted nucleoside), GuNA nucleoside (*e.g.*, R=H in Figure 3 is GuNA[N-H] nucleoside, and R=Me is GuNA[N-Me] nucleoside), or the like. A bicyclic nucleoside having a methyl-

eneoxy(4'-$CH_2$-O-2') bridge is also referred to as LNA nucleoside.

A "non-natural nucleotide" means herein any nucleotide other than a natural nucleotide. For example, it comprises a modified nucleotide and a nucleotide mimic. A "modified nucleotide" means herein a nucleotide having any one or more of a modified sugar moiety, a modified internucleoside linkage, and a modified nucleobase. The term "nucleotide mimic" herein comprises a structure used to substitute a nucleoside and a bond (linkage) at one or more positions in an oligomer compound. Examples of the nucleotide mimic comprise a peptide nucleic acid, and a morpholino nucleic acid (morpholino linked with -N(H)-C(=O)-O- or other non-phosphodiester linkages). The peptide nucleic acid (PNA) is a nucleotide mimic having a main chain in which N-(2-aminoethyl)glycine in place of a sugar is linked with an amide linkage. A nucleic acid strand comprising a non-natural oligonucleotide herein has in many cases preferable properties, such as enhanced cellular uptake, enhanced affinity for a target nucleic acid, increased stability in the presence of a nuclease, and increase in inhibitory activity.

A "modified internucleoside linkage" means herein an internucleoside linkage that has a substitution or any change from a naturally occurring internucleoside linkage (*i.e.,* phosphodiester linkage). A modified internucleoside linkage comprises an internucleoside linkage that comprises a phosphorus atom, and an internucleoside linkage that does not comprise a phosphorus atom. Typical examples of the phosphorus-containing internucleoside linkage comprise, but are not limited to, a phosphodiester linkage, a phosphorothioate linkage, a phosphorodithioate linkage, a phosphotriester bond (a methylphosphotriester linkage and an ethylphosphotriester linkage described in U.S. Pat. No. 5,955,599), an alkylphosphonate linkage (*e.g.*, a methylphosphonate linkage described in U.S. Pat. Nos. 5,264,423 and 5,286,717, and a methoxypropylphosphonate linkage described in WO2015/168172), an alkylthiophosphonate linkage, a methylthiophosphonate linkage, a boranophosphate linkage, an internucleoside linkage containing a cyclic guanidine moiety (*e.g.*, a partial structure represented by the following Formula (I):

[Chem. 1]

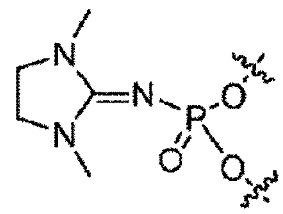

(I)

), an internucleoside linkage containing a guanidine moiety substituted with one to four $C_{1-6}$ alkyl groups (*e.g.,* a tetramethyl guanidine (TMG) moiety) (*e.g.,* a partial structure represented by the following Formula (II):

[Chem. 2]

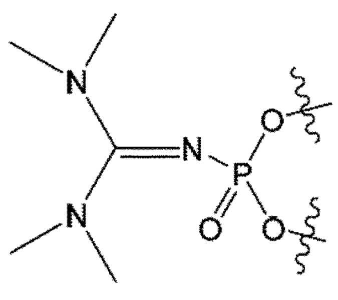

(II)

), and an internucleoside linkage and a phosphoramidate linkage that are to be used for a self-neutralizing nucleic acid (ZON) as described in WO2016/081600. A phosphorothioate linkage refers to an internucleoside linkage in which an unbridged oxygen atom in a phosphodiester linkage is substituted with a sulfur atom. A method for preparing a phosphorus-containing and a phosphorus-free linkage is well known. It is preferable that a modified internucleoside linkage is a linkage having a higher resistance to a nuclease than a naturally occurring internucleoside linkage.

When an internucleoside bond has a chiral center, the internucleoside bond may be chirally controlled. The term "chirally controlled" means that a single diastereomer is present with respect to the chiral center, for example, chirally bound phosphorus. An internucleoside bond chirally controlled may be completely chirally pure, or may have a

high chiral purity, for example, a chiral purity of 90% de, 95% de, 98% de, 99% de, 99.5% de, 99.8% de, 99.9% de, or more. A "chiral purity" herein refers to the proportion of one diastereomer in a diastereomer mixture, is represented as the diastereomeric excess rate (% de), and is defined as (diastereomer of interest - Other diastereomers) / (Total diastereomer) × 100 (%).

For example, the internucleoside bond may be a phosphorothioate bond chirally controlled to an Rp configuration or Sp configuration, an internucleoside bond comprising a guanidine moiety substituted with one to four $C_{1-6}$ alkyl groups (for example, a tetramethyl guanidine (TMG) moiety; see, for example, Alexander A. Lomzov et al., Biochem Biophys Res Commun., 2019, 513 (4), 807-811) (*e.g.*, a partial structure represented by Formula (II)), and/or an internucleoside bond comprising a cyclic guanidine moiety (*e.g.*, a partial structure represented by Formula (I)). A method for preparing an internucleoside bond chirally controlled is publicly known, and a phosphorothioate bond chirally controlled to an Rp configuration or Sp configuration can be synthesized according to, for example, a method described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, or Yohei Nukaga et al., J. Org. Chem. 2012, 77, 7913-7922. A phosphorothioate bond chirally controlled to an Rp configuration or Sp configuration is also publicly known, and is known to produce an effect described in, for example, Naoki Iwamoto et al., Nat. Biotechnol,. 2017, 35(9), 845-851, or Anastasia Khvorova et al., Nat. Biotechnol., 2017, 35(3), 238-248. For example, in one embodiment, a chirally controlled phosphorothioate linkage in the Sp configuration is more stable than those in the Rp configuration and/or chirally controlled ASOs in the Sp configuration promote target RNA cleavage by RNase H1, resulting in a more sustained response *in vivo.* A method for preparing an internucleoside bond comprising a guanidine moiety substituted with one to four $C_{1-6}$ alkyl groups (for example, a TMG moiety) is publicly known, and the internucleoside bond can be synthesized, for example, in accordance with the method described in Alexander A. Lomzov et al., Biochem Biophys Res Commun., 2019, 513 (4), 807-811.

The term "nucleobase" or "base" used herein is a base component (heterocyclic moiety) constituting a nucleic acid. As the component, mainly adenine, guanine, cytosine, thymine, and uracil are known. The "nucleobase" or "base" herein encompasses both of a modified and an unmodified nucleobase (base), unless otherwise specified. Accordingly, a purine base may be any of a modified and an unmodified purine base, unless otherwise specified. In addition, a pyrimidine base may be any of a modified and an unmodified pyrimidine base, unless otherwise specified.

A "modified nucleobase" or a "modified base" means any nucleobase other than adenine, cytosine, guanine, thymine, or uracil. The term "unmodified nucleobase" or "unmodified base" (a natural nucleobase) means adenine (A) and guanine (G), which are purine bases, and thymine (T), cytosine (C), and uracil (U), which are pyrimidine bases. Examples of a modified nucleobase comprise, but are not limited to, 5-methylcytosine, 5-fluorocytosine, 5-bromocytosine, 5-iodocytosine, and *N*4-methylcytosine; *N*6-methyladenine or 8-bromoadenine; 2-thio-thymine; and *N*2-methylguanine or 8-bromoguanine. The modified nucleobase is preferably 5-methylcytosine.

The term "complementary" as used herein refers to the relationship that nucleobases can form via hydrogen bonds so-called Watson-Crick base pairs (natural base pairs) or non-Watson-Crick base pairs (*e.g.*, Hoogsteen base pairs). In the present invention, the antisense oligonucleotide region of the first nucleic acid strand is not necessarily required to be completely complementary to at least a part of a target transcription product (*e.g.*, the transcription product of a target gene), and it is acceptable if the base sequence has at least 70%, preferably at least 80%, and further preferably at least 90% (*e.g.*, 95%, 96%, 97%, 98%, or 99% or more) of complementarity. The antisense oligonucleotide region in the first nucleic acid strand can hybridize to a target transcription product when the base sequence is complementary (typically when the base sequence is complementary to the base sequence of at least a part of the target transcription product). Similarly, the complementary region in the second nucleic acid strand is not necessarily required to be completely complementary to at least a part of the first nucleic acid strand, and it is acceptable if the base sequence has a complementarity of at least 70%, preferably at least 80%, and further preferably at least 90% (*e.g.*, 95%, 96%, 97%, 98%, or 99% or more). The complementary region in the second nucleic acid strand can be annealed when the base sequence of the region is complementary to the base sequence of at least a part of the first nucleic acid strand. Base sequence complementarity can be determined by using a BLAST program or the like. Those skilled in the art can easily determine the conditions (temperature, salt concentration, and the like) under which two strands can anneal or hybridize, taking into account the degree of complementarity between the strands. Furthermore, those skilled in the art can easily design an antisense nucleic acid complementary to a target transcription product, for example, based on information about the base sequence of a target gene.

Hybridization conditions may be variously stringent, for example, low-stringent and high-stringent conditions. Low-stringent conditions may be relatively low temperature and high salt concentration conditions, *e.g.*, 30°C, 2 × SSC, 0.1% SDS. High-stringent conditions may be relatively high temperature and low salt concentration conditions, *e.g.*, 65°C, 0.1 × SSC, 0.1% SDS. The stringency of hybridization can be adjusted by changing conditions such as temperature and salt concentration. In this connection, 1 × SSC contains 150 mM sodium chloride and 15 mM sodium citrate.

A "subject" herein refers to the object to which the double-stranded nucleic acid complex or pharmaceutical composition of the present invention is applied. A subject comprises an individual as well as an organ, a tissue, and a

cell. When the subject is an individual, any animal including a human may be applicable. For example, in addition to a human, a variety of domestic animals, domestic fowls, pets, and laboratory animals are included. Without limitation, the subject may be an individual in need of a decrease in the expression amount of a target transcription product, or an individual in need of treatment or prevention of a disease.

"Body fluid" herein encompasses spinal fluid, interstitial fluid, blood (encompassing serum, plasma, and interstitial fluid), lymph, liquid extract of a tissue or a cell, pleural effusion, sputum, lacrimal fluid, nasal discharge, saliva, urine, and the like.

1-3. Configuration

The double-stranded nucleic acid complex of the present invention comprises a first nucleic acid strand and a second nucleic acid strand. The specific configuration of each nucleic acid strand is described below.

In a double-stranded nucleic acid complex of the present invention, the first nucleic acid strand comprises an artificial nucleic acid, and is capable of hybridizing to at least part of a target gene or a transcription product thereof, and can induce exon skipping, exon inclusion and/or steric blocking to said target gene or transcription product thereof.

The number of artificial nucleic acids in the first nucleic acid strand is not limited, and may be, for example, 1 or more, 2 or more, 3 or more, 4 or more, 5 or more, 6 or more, 7 or more, 8 or more, 9 or more, 10 or more, or 12 or more. The ratio of the artificial nucleic acid(s) in the nucleic acids of the first nucleic acid strand is not limited, and may be, for example, 10% or more, 20% or more, 30% or more, 40% or more, 50% or more, 60% or more, 70% or more, 80% or more, 90% or more, or 100%.

In one embodiment, the artificial nucleic acid(s) comprised in the first nucleic acid strand may comprise any one or more of a morpholino nucleic acid, a 2'-O-methyl-modified nucleoside (2'-OMe-modified nucleoside), 2'-O-methoxyethyl-modified nucleoside (2'-O-MOE-modified nucleoside), a tricyclo-DNA, a peptide nucleic acid, and 2'-O-[2-(*N*-methylcarbamoyl)ethyl]-modified nucleoside (2'-O-MCE-modified nucleoside), and, for example, may comprise any combination thereof. In a further embodiment, the artificial nucleic acid(s) comprised in the first nucleic acid strand consist(s) of a morpholino nucleic acid, a 2'-O-methyl-modified nucleoside, a 2'-O-methoxyethyl-modified nucleoside, a tricyclo-DNA, a peptide nucleic acid, or a 2'-O-[2-(*N*-methylcarbamoyl)ethyl]-modified nucleoside. Herein, a morpholino nucleic acid, a 2'-O-methyl-modified nucleoside, a 2'-O-methoxyethyl-modified nucleoside, a tricyclo-DNA, a peptide nucleic acid, and a 2'-O-[2-(*N*-methylcarbamoyl)ethyl]-modified nucleoside are sometimes collectively referred to as a "morpholino nucleic acid or the like". In one embodiment, the nucleic acids in the first nucleic acid strand consist of morpholino nucleic acids.

In the double-stranded nucleic acid complex of the present invention, the second nucleic acid strand comprises a base sequence complementary to at least part of the first nucleic acid strand, and comprises at least one blocking region which is resistant to a nuclease which is present in a body fluid, and at least one cleavage region which undergoes degradation by a nuclease which is present intracellularly.

A "nuclease which is present in a body fluid" herein refers to a nuclease (for example, RNase or DNase) contained in any body fluid such as blood, spinal fluid, or lymph. Examples of the nuclease contained in blood include RNase A and DNase I.

In addition, a "nuclease which is present intracellularly" herein refers to a nuclease (for example, RNase or DNase) which is present in any cell, preferably, in a target cell. Examples of the nuclease which is present intracellularly include RNase H1, RNase H2, RNase P, RNase L, RNase T2, RNase 2, and DNase II.

A "blocking region" herein refers to a region having resistance to a nuclease which is present in a body fluid. The blocking region may be a region having resistance to a nuclease which is present in any one kind of body fluid, or may be a region having resistance to a nuclease which is present in a plurality of body fluids. In addition, the resistance to cleavage by a nuclease does not need to be complete resistance, and may be such resistance that is increased, compared with a region having the identical base sequence, and comprising an unmodified ribonucleoside.

In one embodiment, the second nucleic acid strand comprises: at least one, for example, 1 to 10, 1 to 5, 1 to 4, 1 to 3, 1 to 2, or 1 blocking region; at least two, for example, 2 to 10, 2 to 5, 3 to 4, 2 to 3, or 2 blocking regions; or at least 3, for example, 3 to 10, 3 to 5, 3 to 4, or 3 blocking regions.

The blocking region consists of one nucleoside or two or more nucleosides linked by an internucleoside bond. The base length of the blocking region is not limited, and may be, for example, 1 to 10 bases in length, 1 to 9 bases in length, 1 to 8 bases in length, 1 to 7 bases in length, 1 to 6 bases in length, 1 to 5 bases in length, 1 to 3 bases in length, or 1 to 2 bases in length.

In one embodiment, the blocking region may comprise at least one or more selected from the group consisting of a deoxyribonucleoside, a 2'-modified nucleoside, a 5'-modified nucleoside, and a bridged nucleoside which comprise a pyrimidine base. Herein, a deoxyribonucleoside, a 2'-modified nucleoside, a 5'-modified nucleoside, and a bridged nucleoside are sometimes collectively referred to as a "deoxyribonucleoside or the like". In a further embodiment, the 2'-modified nucleoside is selected from the group consisting of a 2'-O-methyl-modified nucleoside, a 2'-O-methoxyethyl-modified nucleoside, and a 2'-O-[2-(*N*-methylcarbamoyl)ethyl]-modified nucleoside. In addition, the 5'-modified nucleoside may be a 5'-cp-modified nucleoside, a 5'-methyl-modified nucleoside, or a 5'-dimethyl-modified nucleoside. In addition, the bridged nucleoside is selected from the group consisting of an LNA nucleoside, a 2',4'-$BNA^{NC}$ nucleoside, a cEt BNA nucleoside, an ENA nucleoside, an AmNA nucleoside, a GuNA nucleoside, an scpBNA nucleoside, an scpBNA2 nucleoside, and a BANA3 nucleoside.

In the blocking region, the number of nucleosides which comprise a pyrimidine base, and are each a deoxyribonucleoside or the like is not limited as long as the number is 1 or more. The number may be, for example, 2 or more, 3 or more, 4 or more, 5 or more, 6 or more, 7 or more, 8 or more, 9 or more, or 10 or more, and may be 30 or less, 25 or less, 20 or less, 15 or less, 10 or less, 9 or less, 8 or less, 7 or less, 6 or less, 5 or less, 4 or less, 3 or less, or 2 or less. In the blocking region, the number of nucleosides which comprise a pyrimidine base, and are each a deoxyribonucleoside or the like is, for example, 1 to 20, 1 to 15, 1 to 12, 1 to 10, 1 to 8, or 1 to 6, and may be, for example, 1, 2, 3, 4, 5, 6, 7, 8, 9, or 10.

The blocking region may be completely complementary to part of the first nucleic acid strand, or may have one or more non-complementary bases (mismatch bases). For example, the blocking region may have one to three, one to two, or one non-complementary base relative to part of the first nucleic acid strand. A region composed of a non-complementary base(s) may form a bulge.

A "cleavage region" herein is a region to be degraded by a nuclease which is present intracellularly. The cleavage region may be a region which can be cleaved by one kind of nuclease present in any cell, or may be a region which can be cleaved by a plurality of nucleases present intracellularly. It is desirable that the cleavage region has resistance to a nuclease which is present in a body fluid.

The cleavage region comprises two or more consecutive sugar-unmodified nucleosides. Herein, the "sugar-unmodified nucleoside" means a nucleoside in which the sugar portion of a natural deoxyribonucleoside or a natural ribonucleoside does not contain a sugar modification, such as a substitution at the 2' position and/or any alteration. The cleavage region may comprise at least one modified base and/or modified internucleoside bond. The number of consecutive sugar-unmodified nucleosides may be, for example, 2 or more, 3 or more, 4 or more, 5 or more, or 6 or more, and may be, for example, 2 to 10, 2 to 9, 2 to 8, 2 to 7, 2 to 6, 2 to 5, 2 to 4, or 2 to 3. In one embodiment, the cleavage region comprises one to ten consecutive sugar-unmodified ribonucleosides.

The second nucleic acid strand comprises: at least one, for example, 1 to 10, 1 to 5, 1 to 4, 1 to 3, 1 to 2, or 1 cleavage region; at least two, for example, 2 to 10, 2 to 5, 3 to 4, 2 to 3, or 2 cleavage regions; or at least three, for example, 3 to 10, 3 to 5, 3 to 4, or 3 cleavage regions; wherein the cleavage region is complementary to part of the first nucleic acid strand.

The cleavage region may be completely complementary to part of the first nucleic acid strand, or may have one or more non-complementary bases. For example, the cleavage region may have one to three, one to two, or one non-complementary base relative to part of the first nucleic acid strand. A region composed of a non-complementary base(s) may form a bulge.

In one embodiment, the sugar-unmodified nucleotide in the cleavage region comprises a natural deoxyribonucleoside or a natural ribonucleoside. For example, all the nucleosides comprised in the sugar-unmodified nucleotide are natural deoxyribonucleosides, or part of the nucleosides, for example, one or two nucleosides are natural deoxyribonucleosides. In addition, in the sugar-unmodified nucleotides in the cleavage region, the internucleoside linkage between a deoxyribonucleoside and a deoxyribonucleoside is a phosphodiester bond, and the internucleoside linkage between a deoxyribonucleoside and a ribonucleoside or the internucleoside linkage between a ribonucleoside and a ribonucleoside is a phosphodiester linkage and/or a phosphorothioate bond.

In one embodiment, the cleavage region comprises a sugar-unmodified ribonucleoside comprising a modified or unmodified purine base. The present embodiment can have the effect of maintaining the cleavage activity by a nuclease which is present intracellularly, and simultaneously reducing the cleavage activity by a nuclease which is present in a body fluid such as RNase A, to a low level, compared with a cleavage region that does not comprise a nucleoside comprising a purine base (for example, a cleavage region that consists of nucleosides comprising a pyrimidine base). In a further embodiment, the cleavage region consists of sugar-unmodified ribonucleosides which comprise a modified or unmodified purine base.

In one embodiment, the sugar-unmodified ribonucleoside may be a natural ribonucleoside, a phosphorothioate-internucleoside-bound ribonucleoside, and/or a deoxyribonucleoside, and is preferably a natural ribonucleoside. For example, the sugar-unmodified ribonucleoside comprises a natural ribonucleoside, and all of the sugar-unmodified ribonucleosides may be natural ribonucleosides, or part of them, for example, one or two sugar-unmodified ribonucleosides may be natural ribonucleosides.

In one embodiment, the second nucleic acid strand does not comprise four or more or three or more consecutive natural ribonucleosides comprising a pyrimidine base, and preferably does not comprise two or more consecutive natural ribonucleosides comprising a pyrimidine base.

In a further embodiment, all of the nucleosides that comprise a pyrimidine base in the second nucleic acid

strand may be deoxyribonucleosides, 2'-modified nucleosides, 5'-modified nucleosides, bridged nucleosides, or a combination thereof.

In one embodiment, the second nucleic acid strand may comprise a non-complementary base, and/or one or more insertion sequences and/or deletions, relative to the first nucleic acid strand. The number of non-complementary bases in the second nucleic acid strand is not limited, and may be, for example, 1 to 10, 1 to 9, 1 to 8, 1 to 7, 1 to 6, 1 to 5, 1 to 4, 1 to 3, 1 to 2, or 1 or 2. The base length of the insertion sequence in the second nucleic acid strand is not limited, and may be, for example, 1 to 10, 1 to 9, 1 to 8, 1 to 7, 1 to 6, 1 to 5, 1 to 4, 1 to 3, 1 to 2, or 1 or 2. The base length of consecutive deletions in the second nucleic acid strand is not limited, and may be, for example, 1 to 10, 1 to 9, 1 to 8, 1 to 7, 1 to 6, 1 to 5, 1 to 4, 1 to 3, 1 to 2, or 1 or 2.

The base length of the first nucleic acid strand and the second nucleic acid strand are not particularly limited, and may be at least 8 bases in length, at least 9 bases in length, at least 10 bases in length, at least 11 bases in length, at least 12 bases in length, at least 13 bases in length, at least 14 bases in length, or at least 15 bases in length. Further, the base length of the first nucleic acid strand and the second nucleic acid strand may be 35 bases in length or less, 30 bases in length or less, 25 bases in length or less, 24 bases in length or less, 23 bases in length or less, 22 bases in length or less, 21 bases in length or less, 20 bases in length or less, 19 bases in length or less, 18 bases in length or less, 17 bases in length or less, or 16 bases in length or less. The first nucleic acid strand and the second nucleic acid strand may have identical lengths or different lengths (for example, one of them is shorter or longer by 1 to 3 bases). In one embodiment, the length of the second nucleic acid strand is shorter than the length of the first nucleic acid strand. In this case, the second nucleic acid strand may be bound to the first nucleic acid strand at any position. For example, the second nucleic acid strand may be bound to any of the 5' side region, the central region, and the 3' side region in the first nucleic acid strand. In one embodiment, the second nucleic acid strand may be at least 8 bases in length. The double-stranded structure formed by the first nucleic acid strand and the second nucleic acid strand may comprise a bulge. The length can be determined according to the balance between the strength of the antisense effect and the specificity of the nucleic acid strand to the target, among other factors such as cost, and synthesis yield.

In one embodiment, the second nucleic acid strand may further comprise at least one overhang region located on one or both of the 5' end side and the 3' end side thereof. The term "overhang region" refers to a region adjacent to a region that is in the second nucleic acid strand, and is complementary to the first nucleic acid strand, namely a nucleotide region in the second nucleic acid strand in which the 5' end of the second nucleic acid strand extends beyond the 3' end of the first nucleic acid strand and/or the 3' end of the second nucleic acid strand extends beyond the 5' end of the first nucleic acid strand when the first nucleic acid strand and the second nucleic acid strand are annealed to form a double-stranded structure, or protruding from the double-stranded structure. The overhang region in the second nucleic acid strand may be located at the 5' end side of the complementary region or at the 3' end side. The overhang region in the second nucleic acid strand may be located at the 5' end side and 3' end side of the complementary region.

The length of the overhang region is not limited, and may be 1 to 20 bases in length, and may be, for example, 2 to 15 bases in length, 2 to 12 bases in length, 2 to 10 bases in length, 2 to 8 bases in length, 2 to 6 bases in length, 2 to 5 bases in length, 2 to 4 bases in length, or 2 to 3 bases in length. In addition, the kind of the nucleosides constituting the overhang region is not limited. For example, the nucleosides may be composed of a natural nucleoside (for example, a deoxyribonucleoside) or a non-natural nucleoside (for example, a bridged nucleoside, such as an LNA nucleoside). In addition, all or part of the internucleoside bonds in the overhang region may be modified internucleoside bonds. The modified internucleoside bond may be, for example, a phosphorothioate bond. The overhang region preferably has protein affinity, liposolubility, and/or nuclease resistance, and may be, for example, composed of deoxyribonucleosides or LNA nucleosides linked by a phosphorothioate bond. In this regard, the base sequence of the overhang region may be a sequence unrelated to the base sequence of a target gene.

At least one, at least two (for example, two), at least three, or at least four nucleosides from the end (5' end, 3' end, or both ends) of the second nucleic acid strand may be non-natural nucleosides (modified nucleosides). Modified nucleosides may contain modified sugars and/or modified nucleobases. The modified sugar may be a 2'-modified sugar (e.g., a sugar containing a 2'-O-methyl group). The modified nucleobase may also be 5-methylcytosine.

In one embodiment, the second nucleic acid strand may have 1 to 8, 1 to 7, 1 to 6, 1 to 5, 1 to 4, or 1 to 3 (for example, 1 to 2, or 1) non-complementary bases, 1 to 8, 1 to 7, 1 to 6, 1 to 5, 1 to 4, or 1 to 3 (for example, 1 to 2, or 1) deleted bases, and/or 1 to 20 (for example, 1 to 15, 1 to 12, 1 to 10, 1 to 8, 1 to 6, 1 to 4, 1 to 3, or 1) inserted bases, relative to the first nucleic acid strand, as long as the second nucleic acid strand and the first nucleic acid strand can form a double strand. The sequence region consisting of inserted bases may form a bulge.

The internucleoside bond in the first nucleic acid strand and the second nucleic acid strand may be a naturally occurring internucleoside bond and/or a modified internucleoside bond. Without limitation, at least one, at least two, or at least three internucleoside bonds from an end (5' end, 3' end or both the ends) of the first nucleic acid strand and/or the second nucleic acid strand are preferably modified internucleoside bonds. In this regard, for example, two internucleoside bonds from the end of a nucleic acid strand refers to an internucleoside bond closest to the end of the nucleic acid strand, and an internucleoside bond positioned next thereto on the opposite side to the end of the nucleic acid

strand. Modified internucleoside bonds in the terminal region of a nucleic acid strand are preferred because they can reduce or inhibit undesired degradation of the nucleic acid strand.

In one embodiment, all or part of the internucleoside bonds of the first nucleic acid strand and/or the second nucleic acid strand may be modified internucleoside bonds. In one embodiment, the first nucleic acid strand and/or the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more modified internucleoside bonds. In one embodiment, the first nucleic acid strand and/or the second nucleic acid strand may each comprise modified internucleoside bonds for at least 5%, at least 10%, at least 20%, at least 30%, at least 40%, at least 50%, at least 60%, at least 70%, at least 80%, at least 90%, at least 93%, at least 95%, at least 98%, or 100%. In one embodiment, the modified internucleoside bond may be a phosphorothioate bond or a boranophosphate bond. In one embodiment, when the nucleic acid in the first nucleic acid strand consists of morpholino nucleic acids, all or part of the internucleoside bonds in the first nucleic acid strand may be phosphorodiamidate bonds.

In one embodiment, the first nucleic acid strand and/or the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more chirally controlled internucleoside bonds. In one embodiment, the first nucleic acid strand and/or the second nucleic acid strand may each comprise chirally controlled internucleoside bonds for at least 5%, at least 10%, at least 20%, at least 30%, at least 40%, at least 50%, at least 60%, at least 70%, at least 80%, at least 90%, or more.

In one embodiment, the first nucleic acid strand and/or the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more non-negatively charged internucleoside bonds (preferably neutral internucleoside bonds). In one embodiment, the first nucleic acid strand and/or the second nucleic acid strand may each comprise non-negatively charged internucleoside bonds for at least 5%, at least 10%, at least 20%, at least 30%, at least 40%, at least 50%, at least 60%, at least 70%, at least 80%, at least 90%, or more.

In one embodiment, at least one, at least two, or at least three internucleoside bonds from the 5' end of the second nucleic acid strand may be a modified internucleoside bond(s). At least one, at least two, or at least three internucleoside bonds from the 3' end of the second nucleic acid strand may be a modified internucleoside bond(s), for example, a phosphorothioate bond, an internucleoside bond comprising a guanidine moiety (*e.g.*, a TMG moiety) substituted with one to four $C_{1-6}$ alkyl groups (*e.g.*, a partial structure represented by Formula (II)), and/or an internucleoside bond comprising a cyclic guanidine moiety (*e.g.*, a partial structure represented by Formula (I)). The modified internucleoside bond may be chirally controlled to an Rp configuration or Sp configuration.

At least one (*e.g.*, three) internucleoside linkage from the 3' end of the second nucleic acid strand may be a modified internucleoside linkage such as a phosphorothioate linkage having a high resistance to an RNase. It is preferable that the second nucleic acid strand comprises a modified internucleoside linkage such as a phosphorothioate modification at the 3' end, because the gene inhibition activity of the double-stranded nucleic acid complex is improved.

In one embodiment, a modified internucleoside bond of the first nucleic acid strand and/or the second nucleic acid strand comprises a non-negatively charged (neutral or cationic) internucleoside bond present in neutral form or cationic form respectively at a pH (for example, the physiological pH (approximately 7.4) of a human, the pH of a delivery site (for example, an organelle, cell, tissue, organ, organism, or the like), or the like), compared with the anionic form (for example, $-O-P(O)(O^-)-O-$ (the anionic form of a natural phosphate bond), $-O-P(O)(S^-)-O-$(the anionic form of a phosphorothioate bond), or the like). In one embodiment, the modified internucleoside bond of the first nucleic acid strand and/or the second nucleic acid strand comprises a neutral internucleoside bond. In one embodiment, the modified internucleoside bond of the first nucleic acid strand and/or the second nucleic acid strand comprises a cationic internucleoside bond. In one embodiment, a non-negatively charged internucleoside bond (for example, a neutral internucleoside bond), when in the neutral form thereof, does not have a moiety the pKa of which is less than 8, less than 9, less than 10, less than 11, less than 12, less than 13, or less than 14. In one embodiment, the non-negatively charged internucleoside bond is, for example, an internucleoside bond or the like to be used for a methylphosphonate bond described in U.S. Pat. Nos. 5,264,423 and 5,286,717, a methylphosphotriester bond and an ethylphosphotriester bond described in U.S. Pat. No. 5,955,599, a methoxypropylphosphonate bond described in WO2015/168172, and a self-neutralizing nucleic acid (ZON) described in WO2016/081600. In one embodiment, the non-negatively charged internucleoside bond comprises a triazole moiety or an alkyne moiety. In one embodiment, the non-negatively charged internucleoside bond comprises a cyclic guanidine moiety and/or a guanidine moiety (preferably a TMG moiety) substituted with one to four $C_{1-6}$ alkyl groups. In one embodiment, the modified internucleoside bond comprising a cyclic guanidine moiety has a partial structure represented by Formula (I). In one embodiment, the guanidine moiety substituted with one to four $C_{1-6}$ alkyl groups has a partial structure represented by Formula (II). In one embodiment, the neutral internucleoside bond comprising a cyclic guanidine moiety and/or a guanidine moiety substituted with one to four $C_{1-6}$ alkyl groups is chirally controlled. In one embodiment, the present disclosure relates to a composition comprising an oligonucleotide comprising at least one neutral internucleoside bond and at least one phosphorothioate internucleoside bond. Without wishing to be bound by any specific theory, but at least in some cases, the neutral internucleotide bond can improve characteristics

and/or activity, for example, improve delivery, improve resistance to exonuclease and endonuclease, improve cellular uptake, improve endosomal escape, and/or improve nucleic uptake, compared with an equivalent nucleic acid comprising no neutral internucleotide bond.

In one embodiment, the second nucleic acid strand may be bound to a ligand (herein sometimes referred to as a binder). Examples of the ligand include a small molecule (low-molecular weight ligand), a middle molecule (middle molecular weight ligand), a macromolecule (high molecular weight ligand), a peptide (peptide ligand), a lipid (lipid ligand), and an aptamer (for example, a nucleic acid aptamer).

A "peptide" herein refers to an amino acid polymer having one or more peptide bonds. The "peptide" is not limited by the number of amino acid residues comprised in the peptide. Accordingly, the "peptide" encompasses an oligopeptide comprising several amino acid residues, such as a dipeptide or a tripeptide, and a polypeptide (protein) comprising many amino acid residues. The peptide can be a linear, branched, or cyclic peptide.

The peptide ligand may be bound to a molecule present on the surface of a cell, in a cell, or in a body fluid.

In one embodiment, the peptide may be an antibody or an active fragment thereof. Examples of the antibody include a monoclonal antibody, a polyclonal antibody, a recombinant antibody such as a chimeric antibody or a humanized antibody, Fab, $F(ab')_2$, Fab', VHH, and the like. Examples of the active fragment of an antibody include an scFv (single chain Fragment of variable region: single chain antibody), a diabody, a triabody, a tetrabody, or the like.

Examples of the lipid (lipid ligand) include, but are not limited to, tocopherol, cholesterol, fatty acid, phospholipid, and analogs thereof; folic acid, vitamin C, vitamin B1, vitamin B2; estradiol, androstane, and analogs thereof; steroid and an analog thereof; ligand of LDLR, SRBI or LRP1/2; FK-506, and cyclosporine; lipids described in PCT/JP2019/12077, PCT/JP2019/10392, and PCT/JP2020/035117. In addition, the lipid (lipid ligand) may be a tocopherol or an analog thereof and/or a cholesterol or an analog thereof, a substituted or unsubstituted $C_{1-30}$ alkyl group, a substituted or unsubstituted $C_{2-30}$ alkenyl group, or a substituted or unsubstituted $C_{1-30}$ alkoxy group.

"Tocopherol" is herein a methylated derivative of tocorol which is a liposoluble vitamin (vitamin E) having a cyclic structure called chroman. Tocorol has a strong antioxidant effect, and therefore functions *in vivo* as an antioxidant substance to scavenge free radicals produced by metabolism and protect cells from damage.

A plurality of different types of tocopherol consisting of α-tocopherol, β-tocopherol, γ-tocopherol, and δ-tocopherol are known based on the position of the methyl group bound to chroman. A tocopherol herein may be any types of tocopherol. In addition, examples of the analog of tocopherol comprise various unsaturated analogs of tocopherol, such as α-tocotrienol, β-tocotrienol, γ-tocotrienol, and δ-tocotrienol. Preferably, tocopherol is α-tocopherol.

"Cholesterol" is herein a kind of sterol, also called steroid alcohol, which is especially abundant in animals. Cholesterol exerts an important function in the metabolic process *in vivo,* and in animal cells, it is also a major constituent of the membrane system of a cell, together with phospholipid. In addition, the cholesterol analog refers to various cholesterol metabolism products and their analogs, which are alcohols having a sterol backbone. Examples thereof include, but are not limited to, cholestanol, lanosterol, cerebrosterol, dehydrocholesterol, and coprostanol.

An "analog" herein refers to a compound having a similar structure and property having the same or a similar basic backbone. The analog comprises, for example, a biosynthetic intermediate, a metabolism product, and a compound having a substituent. Those skilled in the art can determine whether or not a compound is an analog of another compound based on common general technical knowledge.

The cholesterol analog refers to various cholesterol metabolism products and their analogs, which are alcohols having a sterol backbone. Examples thereof include, but are not limited to, cholestanol, lanosterol, cerebrosterol, dehydrocholesterol, and coprostanol.

In one embodiment, the second nucleic acid strand may be bound to tocopherol or cholesterol, or an analog thereof. The second nucleic acid strand bound to cholesterol or an analog thereof may have a group represented by the following Formula (III).

[Chem. 3]

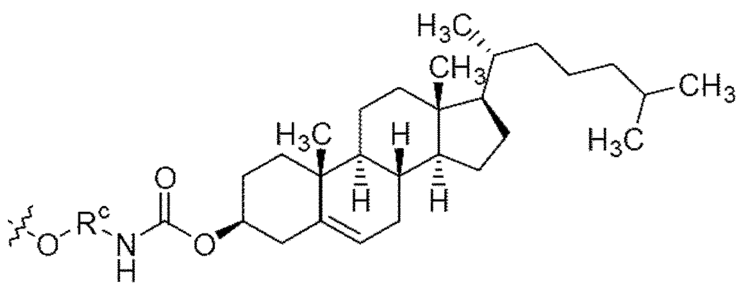

(III)

,

wherein, $R^c$ represents a $C_{4-18}$, preferably $C_{5-16}$, alkylene group optionally having a substituent (here, the substituent is a halogen atom or a $C_{1-3}$ alkyl group optionally substituted with a hydroxy group, in which such an alkyl group is, for example, a hydroxymethyl group; and in the alkylene group, a carbon atom not mutually adjacent to another carbon atom may be substituted with an oxygen atom).

$R^c$ is not limited, and may be $-(CH_2)_3-O-(CH_2)_2-O-(CH_2)_2-O-(CH_2)_2-O-(CH_2)_2-$, $-(CH_2)_3-O-(CH_2)_2-O-(CH_2)_2-O-(CH_2)_2-O-CH_2-CH(CH_2OH)-$, or $-(CH_2)_6-$.

A group represented by the above-described Formula (III) can be bound to the 5' end or 3' end of the second nucleic acid strand via a phosphoester bond.

The ligand that is cholesterol, an analog thereof, or the like may be bound to any of the 5' end, the 3' end, and both ends of the second nucleic acid strand. In addition, the ligand that is cholesterol, an analog thereof, or the like may be bound to a nucleotide at an interior position in the second nucleic acid strand.

When the second nucleic acid strand comprises a plurality of cholesterols or analogs thereof, the cholesterols or analogs may be identical or different. In this case, for example, cholesterol and another cholesterol analog are one each bound to the 5' end and 3' end of the second nucleic acid strand respectively. With regards to the binding position, the cholesterol or an analog thereof may be bound to a plurality of positions in the second nucleic acid strand, and/or may be bound, in the form of one group, to one position. One cholesterol or an analog thereof may be linked to each of the 5' end and 3' end of the second nucleic acid strand.

The bond between the second nucleic acid strand and the ligand may be a direct bond or an indirect bond that is mediated by another substance.

When the second nucleic acid strand and a ligand are directly bound, the ligand can be bound to the second nucleic acid strand, for example, via a covalent bond, an ionic bond, a hydrogen bond, or the like. A covalent bond is preferable in view of the capability to form a more stable bond.

In one embodiment, the second nucleic acid strand is not bound to a ligand. Herein, being "not bound to a ligand" refers to being not bound to a ligand such as tocopherol or cholesterol. In a further embodiment, the double-stranded nucleic acid complex of the present invention is not bound to a ligand, that is, neither the first nucleic acid strand nor the second nucleic acid strand is bound to a ligand.

When the second nucleic acid strand and cholesterol or an analog thereof are indirectly bound to each other, both of them may be bound via a linking group (herein often referred to as a "linker"). The linker may be any one of a cleavable linker and an uncleavable linker.

A "cleavable linker" refers to a linker that can be cleaved under physiological conditions, for example, in a cell or in an animal body (*e.g.*, in a human body). A cleavable linker is selectively cleaved by an endogenous enzyme such as a nuclease. Examples of a cleavable linker comprise, but are not limited to, an amide, an ester, one or both esters of a phosphodiester, a phosphoester, a carbamate, and a disulfide bond, as well as a natural DNA linker. In one example, cholesterol or an analog thereof may be linked via a disulfide bond.

An "uncleavable linker" refers to a linker that is not cleaved under physiological conditions, for example, in a cell or in an animal body (*e.g.*, in a human body). Examples of an uncleavable linker comprise, but are not limited to, a phosphorothioate linkage, modified or unmodified deoxyribonucleosides linked by a phosphorothioate linkage, and a linker consisting of modified or unmodified ribonucleosides. There is no particular restriction on the chain length, when a linker is a nucleic acid such as DNA, or an oligonucleotide, however it may be usually from 2 to 20 bases in length, from 3 to 10 bases in length, or from 4 to 6 bases in length.

Specific examples of the linker comprise a linker represented by the following Formula (IV).

[Chem. 4]

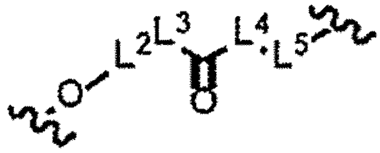

(IV)

,

wherein $L^2$ represents a substituted or unsubstituted $C_1$-$C_{12}$ alkylene group (for example, propylene, hexylene, dodecylene), a substituted or unsubstituted $C_3$-$C_8$ cycloalkylene group (for example, cyclohexylene), $-(CH_2)_2-O-(CH_2)_2-O-(CH_2)_2-O-(CH_2)_3-$, $-(CH_2)_2-O-(CH_2)_2-O-(CH_2)_2-O-(CH_2)_2-O-(CH_2)_3-$, or $CH(CH_2-OH)-CH_2-O-(CH_2)_2-O-(CH_2)_2-O-(CH_2)_2-O-(CH_2)_3-$, $L^3$ represents -NH- or a bond, $L^4$ represents a substituted or unsubstituted $C_1$-$C_{12}$ alkylene group (for example, ethylene, pentylene, heptylene, or undecylene), a substituted or unsubstituted $C_{3-8}$ cycloalkylene group (for example, cyclohexylene), $-(CH_2)_2-[O-(CH_2)_2]_m-$, or a bond, wherein m is an

integer of 1 to 25, $L^5$ represents -NH-(C=O)-, -(C=O)-, or a bond (here, the substitution is preferably carried out by a halogen atom.

In one embodiment, regarding a linker represented by Formula (IV), $L^2$ is unsubstituted $C_3$-$C_6$ alkylene group (for example, propylene, hexylene), $-(CH_2)_2-O-(CH_2)_2-O-(CH_2)_2-O-(CH_2)_3-$, or $-(CH_2)_2-O-(CH_2)_2-O-(CH_2)_2-O-(CH_2)_2-O-(CH_2)_3-$, $L^3$ is -NH-, and $L^4$ and $L^5$ are bonds.

In addition, other specific examples of the linker comprise a linker represented by the following Formula (V).

[Chem. 5]

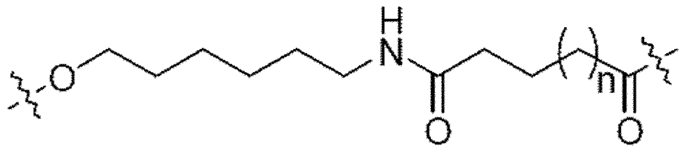

(V),

wherein, n represents 0 or 1.

The first nucleic acid strand and/or the second nucleic acid strand (preferably the second nucleic acid strand) may further comprise at least one functional moiety bound to a polynucleotide constituting the nucleic acid strand. A "functional moiety" refers to a moiety that provides a desired function to the double-stranded nucleic acid complex and/or the nucleic acid strand to which the functional moiety is bound. Examples of the desired function include a labeling function, a purification function, and the like. Examples of a moiety that provides a labeling function include compounds such as a fluorescent protein, and luciferase. In addition, examples of a moiety that provides a purifying function include compounds such as biotin, avidin, His-tag peptide, GST-tag peptide, and FLAG-tag peptide. In one embodiment, a molecule having an activity for delivery of a double-stranded nucleic acid complex in an embodiment to a target site is preferably bound as a functional moiety to the second nucleic acid strand, from the viewpoint that the first nucleic acid strand is efficiently delivered to a target site at a high specificity and expression of a target gene is very effectively suppressed by the nucleic acid. Examples of a moiety for providing a delivery function to a target include lipid, antibody, aptamer, and a ligand to a particular receptor. In one embodiment, the first nucleic acid strand and/or the second nucleic acid strand (preferably the second nucleic acid strand) is bound to a functional moiety. The linkage between the second nucleic acid strand and the functional moiety may be a direct linkage or an indirect linkage via another substance, and in some embodiments, it is preferable that the second nucleic acid strand and the functional moiety are directly bound by covalent bonding, ionic bonding, hydrogen bonding, or the like, and from the viewpoint of obtaining a more stable bond, covalent bonding is more preferable.

The first nucleic acid strand and the second nucleic acid strand may be linked via a linker. In this case, the first nucleic acid strand and the second nucleic acid strand can be linked via a linker to form a single strand. The double-stranded nucleic acid complex in this case can be referred to as a hinge nucleic acid, a single-stranded HDO, an ssHDO, or the like. However, even in that case, the functional region has the same configuration as the double-stranded nucleic acid complex, and therefore such a single-stranded nucleic acid is herein also encompassed as an embodiment of the double-stranded nucleic acid complex of the present invention. The linker can be any polymer. Examples thereof include a polynucleotide, polypeptide, and alkylene. Specifically, it can be composed of a natural nucleotide such as DNA, and RNA, or a non-natural nucleotide such as a peptide nucleic acid and a morpholino nucleic acid. When a linker consists of a nucleic acid, the chain length of a linker may be at least one base, or a chain length of from 3 to 10 bases or from 4 to 6 bases. It is preferably 4 bases in length. The position of the linker can be either on the 5' side or the 3' side of the first nucleic acid strand. For example, in the case of a configuration in which cholesterol or an analog thereof is bound to the 5' side of the second nucleic acid strand, the 5' end of the first nucleic acid strand and the 3' end of the second nucleic acid strand are linked via a linker. The linker may be cleavable or uncleavable.

In the double-stranded nucleic acid complex of the present invention, the antisense effect on the target transcription product of the first nucleic acid strand can be measured by a method publicly known in the art. For example, after introducing a double-stranded nucleic acid complex into a cell and the like, it can be measured using a publicly known technique such as Northern blotting, quantitative PCR, or Western blotting. By measuring the expression level of a target gene or the level of a target transcription product in specific tissues (*e.g.*, the amount of mRNA, the amount of RNA such as microRNA, the amount of cDNA, and the amount of protein), it can be judged whether or not the target gene expression is suppressed by the double-stranded nucleic acid complex in these sites. For example, regarding exon skipping, the effect can be determined by comparing a product produced by exon skipping with a product produced without exon skipping.

An exemplary embodiment of the double-stranded nucleic acid complex of the present invention has been

described above, however, the double-stranded nucleic acid complex of the present invention is not limited to the above exemplary embodiment.

1-4. Method for producing a double-stranded nucleic acid complex

Those skilled in the art can produce the double-stranded nucleic acid complex of the present invention by appropriately selecting a publicly known method. Usually, without limitation, firstly each of the first nucleic acid strand and the second nucleic acid strand that constitute a double-stranded nucleic acid complex is designed and prepared. For example, the first nucleic acid strand is designed based on the information on the base sequence of the target transcription product (*e.g.*, the base sequence of the target gene), and the second nucleic acid strand is designed as a complementary strand thereto. Then, based on the information on the designed base sequences, each nucleic acid strand is synthesized using a commercially available automatic nucleic acid synthesizer, such as that from GE Healthcare, Thermo Fisher Scientific, or Beckman Coulter. Thereafter the prepared oligonucleotides may be purified using a reverse-phase column or the like.

In the case of a double-stranded nucleic acid complex to which a functional moiety is bound, a first nucleic acid strand may be produced according to the above method. Meanwhile, with respect to a second nucleic acid strand to which a functional moiety is bound, it may be produced by performing the aforedescribed synthesis and purification using a nucleic acid species to which a functional moiety has been bound in advance. For example, a second nucleic acid strand may be produced by performing the aforedescribed synthesis and purification using a nucleic acid species to which cholesterol or an analog thereof has been bound in advance. Alternatively, cholesterol or an analog thereof may be joined by a publicly known method to a second nucleic acid strand produced by performing the aforedescribed synthesis and purification. After preparation of each nucleic acid strand, a double-stranded nucleic acid complex to which the functional moiety of interest is bound can be produced by performing annealing for the first nucleic acid strand and the second nucleic acid strand. Specifically, the nucleic acids are mixed in an appropriate buffer solution to be denatured at about 90°C to 98°C for several minutes (*e.g.*, 5 min), and then the nucleic acids are annealed in a range of about 30°C to 70°C for about 1 to 8 hours to yield a double-stranded nucleic acid complex of the present invention. The method for linking a functional moiety to a nucleic acid is well known in the art. Further, a nucleic acid strand can be obtained by ordering from various manufacturers (*e.g.*, GeneDesign Inc.) by specifying the base sequence and the modification site and type.

1-5. Application of double-stranded nucleic acid complex

In one embodiment, the double-stranded nucleic acid complex of the present invention may be for at least one of the following actions in a subject: the action of suppressing or increasing the expression level of a transcription product or a translation product of a target gene; the action of inhibiting a function of a transcription product or a translation product of a target gene; the action of regulating RNA splicing; and applications for the action of inhibiting binding of a target gene to a protein, such as exon skipping. For example, the double-stranded nucleic acid complex of the present invention may be for at least one of the following actions: exon skipping, exon inclusion, steric blocking, and increasing RNA expression. The double-stranded nucleic acid complex of the present invention may be used for providing the above-described action in a particular tissue, *e.g.*, brain, spinal cord, kidney, liver, lung, intestinal tract, spleen, adrenal gland, eye, retina, skin, or peripheral nerves, such as a brain. The brain may be cerebrum, diencephalon, brain stem, or cerebellum, *e.g.*, one or more of the following: cerebrum (*e.g.*, cerebral cortex), brain stem, cerebellum, hippocampus, and striatum. The double-stranded nucleic acid complex of the present invention may be used to provide the above-described action in a muscle tissue, including heart muscle and skeletal muscle.

In one embodiment, the double-stranded nucleic acid complex of the present invention is for disease or prevention. The disease may be skeletal muscle dysfunction or cardiac dysfunction. Examples of a disease include muscular dystrophies (Duchenne muscular dystrophy, myotonic dystrophy type 1 (DM1), Fukuyama muscular dystrophy, facioscapulohumeral muscular dystrophy, limb-girdle muscular dystrophy, or the like), congenital myopathies, primary age-related tauopathies (PART), Alzheimer's disease (AD), progressive supranuclear palsy (PSP), corticobasal degeneration/corticobasal syndrome (CBD), Pick's disease, frontotemporal dementia, neuroinclusion body disease, spinal muscular atrophy (SMA), amyotrophic lateral sclerosis (ALS), Huntington's disease, hereditary spinocerebellar ataxia (SCA), multiple system atrophy, hereditary spastic paraplegia, multiple sclerosis, stroke, epilepsy, and encephalitis.

1-6. Effects

With a double-stranded nucleic acid complex of the present invention, a region composed of nucleosides having resistance to a nuclease present in a body fluid, such as RNase A, functions as a stable region (blocking region) in the second nucleic acid strand, whereby the double-stranded nucleic acid complex of the present invention can avoid

degradation, and be stabilized in the blood, in the spinal fluid, or in the like. This results in enabling the first nucleic acid strand to be prevented from being released from the double-stranded nucleic acid complex in the blood, in the spinal fluid, or in the like, and to be prevented from being renally excreted.

On the other hand, after the double-stranded nucleic acid complex of the present invention transitions into a cell, the first nucleic acid strand is released from the double-stranded nucleic acid complex, and enabled to function for a target transcription product or the like. The release of the first nucleic acid strand from the double-stranded nucleic acid complex can be promoted by allowing the cleavage region in the second nucleic acid strand to be cleaved by a nucleic acid-degrading enzyme in a cell.

## 2. Pharmaceutical composition

### 2-1. Overview

A second aspect of the present invention is a pharmaceutical composition. The pharmaceutical composition of the present invention comprises the double-stranded nucleic acid complex described in said first aspect as an active ingredient.

Each component that a pharmaceutical composition of the present invention may comprise will be described in detail below.

### 2-2. Configuration

#### 2-2-1. Active ingredient

The pharmaceutical composition of the present invention comprises as an active ingredient at least a double-stranded nucleic acid complex described in the first aspect. A pharmaceutical composition of the present invention may comprise only one kind of double-stranded nucleic acid complex, or may comprise two or more kinds thereof.

The amount (content) of the double-stranded nucleic acid complex in a pharmaceutical composition varies depending on the kind of the double-stranded nucleic acid complex, the delivery site, the dosage form of the pharmaceutical composition, the dose of the pharmaceutical composition, and the kind of a carrier described below. Therefore, it may be determined as appropriate by taking the respective conditions into consideration. Usually, it may be adjusted so that an effective amount of the double-stranded nucleic acid complex is comprised in a single dose of the pharmaceutical composition. An "effective amount" refers to an amount that is necessary for the double-stranded nucleic acid complex to function as an active ingredient, and to an amount that has little or no adverse side effect on a living body to which the amount is applied. This effective amount can vary depending on various conditions such as information on the subject, the administration route, and number of administrations. Ultimately, it may be determined by the judgment of a physician, veterinarian, pharmacist, or the like. "Information on the subject" is various information on an individual of the living body to which the pharmaceutical composition is applied. For example, when the subject is a human, it comprises age, body weight, gender, dietary habit, health status, stage of progression or grade of severity of the disease, drug sensitivity, and presence of a drug for use in combination.

#### 2-2-2. Carrier

The pharmaceutical composition of the present invention may comprise a pharmaceutically acceptable carrier. A "pharmaceutically acceptable carrier" refers to an additive commonly used in the field of pharmaceutical preparation. Examples thereof include a solvent, a vegetable oil, a base, an emulsifier, a suspending agent, a surfactant, a pH adjuster, a stabilizer, a seasoning, a flavor, an excipient, a vehicle, a preservative, a binder, a diluent, an isotonizing agent, a sedative, a bulking agent, a disintegrating agent, a buffering agent, a coating agent, a lubricant, a colorant, a sweetener, a thickener, a corrective agent, a dissolution aid, and other additives.

The solvent may be any of, for example, water or other pharmaceutically acceptable aqueous solution, and a pharmaceutically acceptable organic solvent. Examples of an aqueous solution comprise a physiological saline, an isotonic solution containing glucose or another additive, a phosphate-buffered saline, and a sodium acetate buffer solution. Examples of the additive comprise D-sorbitol, D-mannose, D-mannitol, sodium chloride, and further a nonionic surfactant at a low concentration, and polyoxyethylene sorbitan fatty acid ester.

The above carrier is used to avoid or decrease degradation of the double-stranded nucleic acid complex, which is an active ingredient, *in vivo* by an enzyme and the like, and additionally to facilitate formulation or administration, and to maintain the dosage form and drug efficacy. Therefore, it may be used as appropriate and as needed.

2-2-3. Dosage form

There is no particular restriction on the dosage form of the pharmaceutical composition of the present invention as long as the double-stranded nucleic acid complex described in the first aspect, which is an active ingredient, is delivered to a target site without being inactivated by degradation or the like, and the pharmacological effect (an antisense effect on the expression of a target gene) of the active ingredient can be produced *in vivo.*

The specific dosage form varies depending on the administration method and/or medication conditions. The administration methods can be broadly classified into parenteral administration and oral administration, and the dosage form appropriate for the respective administration methods can be selected.

When the administration method is parenteral administration, the preferred dosage form is liquid formulation which can be administered directly to the target site, or administered systemically via the circulatory system. Examples of the liquid formulation comprise an injectable. An injectable can be formulated by mixing in an appropriate combination with the aforedescribed excipient, elixir, emulsifier, suspending agent, surfactant, stabilizer, pH adjuster, *etc.* in the form of a unit dose required according to the generally approved pharmaceutical practices. In addition, it may be ointment, plaster, cataplasm, transdermal patch, lotion, inhalant, aerosol, eye drop, and suppository.

When the administration method is oral administration, the preferred dosage form may be a solid preparation or a liquid formulation. Examples include a tablet, capsule, drop, lozenge, pill, granule, dusting powder, powder, oral liquid preparation, emulsion, syrup, pellet, lingusorbs, peptizer, buccal, paste, suspending agent, elixir, coating agent, ointment, plaster, cataplasm, transdermal patch, lotion, inhalant, aerosol, eye drop, injectable, and suppository. In the case of a solid preparation, if necessary, it may take a dosage form with a coating as publicly known in the art, such as a sugar-coated tablet, a gelatin-coated tablet, an enteric-coated tablet, a film-coated tablets, a double layer tablet, and a multilayer tablet.

There is no particular restriction on the specific shape and size of each of the above-mentioned dosage forms, as long as the respective dosage forms are within the ranges of dosage forms publicly known in the art. As for the manufacturing method of the pharmaceutical composition of the present invention, it may be formulated according to the common procedure in the art.

In a specific embodiment, a double-stranded nucleic acid complex of the present invention has excellent quality as a pharmaceutical product having the following: excellent solubility in water, the second fluid for the dissolution test in accordance with the Japanese Pharmacopoeia, or the second fluid for the disintegration test in accordance with the Japanese Pharmacopoeia; excellent pharmacokinetics (for example, the drug half-life in the blood, intracerebral transitivity, metabolic stability, and CYP inhibition); low toxicity (for example, superior as a pharmaceutical product from the viewpoint of acute toxicity, chronic toxicity, genotoxicity, reproductive toxicity, cardiotoxicity, drug interaction, carcinogenicity, light toxicity, and the like); less side effects (for example, suppression of sedation and avoidance of laminar necrosis); and the like.

2-3. Dosing form and dose

Herein there is no particular restriction on the preferable dosing form of a pharmaceutical composition. For example, the dosing from may be oral or parenteral administration. Specific examples of the parenteral administration include intramuscular administration, intravenous administration, intraarterial administration, intraperitoneal administration, subcutaneous administration (comprising implantable continuous subcutaneous administration), intradermal administration, trachea/bronchial administration, rectal administration, administration by blood transfusion, intraventricular administration, intrathecal administration (e.g., lumbar puncture), transnasal administration, intraocular administration, inhalation administration, and intramuscular administration.

When a pharmaceutical composition is applied by administration or ingestion, the administered amount or ingested amount may be, for example, from 0.00001 mg/kg/day to 10000 mg/kg/day, or from 0.001 mg/kg/day to 100 mg/kg/day for the double-stranded nucleic acid complex contained in the pharmaceutical composition. A pharmaceutical composition may be applied by single-dose administration or multiple dose administration. In the case of multiple dose administration, it may be administered daily or at appropriate time intervals (*e.g.*, at intervals of 1 day, 2 days, 3 days, 1 week, 2 weeks, or 1 month), for example, for 2 to 20 times. A single dose of the double-stranded nucleic acid complex described above may be, for example, 0.001 mg/kg or more, 0.005 mg/kg or more, 0.01 mg/kg or more, 0.25 mg/kg or more, 0.5 mg/kg or more, 1 mg/kg or more, 2.5 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount in the range of from 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

The double-stranded nucleic acid complex of the present invention may be administered twice a week for total

four times at a dose of from 0.01 to 10 mg/kg (e.g., about 6.25 mg/kg). Alternatively, the double-stranded nucleic acid complex may be administered once or twice a week for total two to four times, for example at a frequency of twice a week for total two times, at a dose of from 0.05 to 30 mg/kg (*e.g.*, about 25 mg/kg). By adopting such a dosing regimen (divided administration), the toxicity can be lowered (*e.g.*, avoidance of platelet reduction) compared to a single-dose administration at a higher dose, and the stress to the subject can be reduced.

Even when the pharmaceutical composition is repeatedly administered, its inhibitory effect can be produced additively in a cell. In the case of repeated administration, the efficacy can be improved with certain administration intervals (*e.g.*, half a day or longer).

In one embodiment, a pharmaceutical composition of the present aspect is intraventricularly administered or intrathecally administered. When a pharmaceutical composition of the present aspect is intraventricularly administered or intrathecally administered, the pharmaceutical composition may be administered to a monkey or a human in an amount of 0.01 mg or more, 0.1 mg or more, or 1 mg or more, for example, 2 mg or more, 3 mg or more, 4 mg or more, 5 mg or more, 10 mg or more, 20 mg or more, 30 mg or more, 40 mg or more, 50 mg or more, 75 mg or more, 100 mg or more, 200 mg or more, 300 mg or more, 400 mg or more, or 500 mg or more, or may be administered in an amount of 0.01 mg to 1000 mg, 0.1 mg to 200 mg, or 1 mg to 20 mg. The pharmaceutical composition may be administered to a mouse in an amount of 1 μg or more.

In one embodiment, a pharmaceutical composition of the present aspect is intravenously administered or subcutaneously administered. When a pharmaceutical composition of the present aspect is intravenously administered or subcutaneously administered, the pharmaceutical composition may be administered in an amount of 0.01 mg/kg or more, 0.1 mg/kg or more, or 1 mg/kg or more, for example, 2 mg/kg or more, 3 mg/kg or more, 4 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more, or may be administered in an amount of 0.01 mg/kg to 1000 mg/kg, 0.1 mg/kg to 100 mg/kg, or 1 mg/kg to 10 mg/kg.

2-4. Disease as subject of application

A disease to which the pharmaceutical composition is applicable is not limited. A disease to which the pharmaceutical composition is applicable may be a disease which may be related to a gene for which the antisense effect of a double-stranded nucleic acid complex of the present invention can suppress or increase the expression amount of a transcription product or translation product or can inhibit the function of a transcription product or translation product of the gene, or can induce steric blocking, splicing switching, RNA editing, exon skipping, or exon inclusion. The disease may be, for example, a neural disease, central nervous system disease, metabolic disease, tumor, infection, skeletal muscle dysfunction or cardiac dysfunction, brain disorder, spinal cord disorder, or peripheral nerve disorder. Specific examples of the disease are as described in "1-5. Application of double-stranded nucleic acid complex".

2-5. Effects

A pharmaceutical composition of the present invention has excellent stability in the blood and/or in the spinal fluid, and thus, can avoid reduction of effects due to degradation or renal excretion before the transition to a target cell. Therefore, even when a pharmaceutical composition of the present invention is administered systemically by intravenous administration or the like, the pharmaceutical composition does not lose efficacy before reaching a target site, and can provide a high antisense effect.

A pharmaceutical composition of the present invention can increase RNA expression or reduce RNA expression by exon skipping, exon inclusion and/or steric blocking, or regulate splice variants. Regulating splice variants means controlling the ratios at which a plurality of splice variants are produced by alternative splicing.

In addition, provided is a method for treating and/or preventing a disease such as a central nervous system disease, comprising administering the above-described double-stranded nucleic acid complex or pharmaceutical composition to a subject.

In addition, provided is use of a double-stranded nucleic acid complex of the present invention in the production of a medicine for treating and/or preventing a disease.

Examples

The present invention will be described below in more detail by means of Examples. However, the technical scope of the present invention is not limited to these Examples.

<Example 1: Exon skipping effect by heteronucleic acid PMO>

(Purpose)

In a double-stranded nucleic acid complex which consists of an antisense oligonucleotide (ASO/phosphorodiamidate morpholino oligomer (hereafter, also referred to as "PMO")) targeting the exon 23/intron 23 boundary region in the dystrophin gene of an mdx mouse (Duchenne muscular dystrophy model mouse) for exon skipping and a complementary strand, a ligand and a nucleic acid modification are introduced into the complementary strand, and the effect on the exon skipping efficiency in the muscle of the whole body is evaluated.

(Method)

(1) Preparation of nucleic acids

As an antisense oligonucleotide (ASO), a 25 mer PMO having a base sequence complementary to the 83803536 to 83803512 positions (complementary to the positions 82847142 to 82847118 of GenBank accession numbers: NC_000086.8) of the dystrophin pre-mRNA (GenBank accession number: NC_000086.7) of mice was used. This PMO targets the exon 23/intron 23 of the dystrophin pre-mRNA, and all of 25 mer is composed of morpholino, and all internucleoside bonds are phosphorodiamidate bonds.

By annealing this morpholino nucleic acid (first nucleic acid strand) with a second nucleic acid strand (Toc-cRNA or Chol-cRNA) that is a tocopherol- or cholesterol-conjugated complementary strand, a tocopherol-conjugated heteroduplex oligonucleotide (Toc-HDO) or a cholesterol-conjugated heteroduplex oligonucleotide (Chol-HDO), which is a double-stranded nucleic acid agent, was prepared. Hereinafter, these Toc-HDO and Chol-HDO are referred to as "Toc-HDO (default)" and "Chol-HDO (default)" respectively.

Furthermore, a double-stranded complex in which cytosine RNA and uracil RNA in the central part of the complementary strand in the above Chol-HDO (default) are substituted with a 2'-O-Me RNA (hereinafter referred to as "Chol-HDO (CU-OMe)") was prepared. In addition, a double-stranded complex in which cytosine RNA and uracil RNA are substituted with DNA (cytosine DNA and thymine DNA, respectively) (hereinafter referred to as "Chol-HDO (CT-DNA)") was prepared.

The first nucleic acid strand was mixed with the second nucleic acid strand in equimolar amounts, the solution was heated at 95°C for 5 min, then cooled to 37°C and maintained for 1 hour allowing the nucleic acid strand to anneal, thereby preparing the above-described double-stranded nucleic acid agent. The annealed nucleic acids were stored at 4°C or on ice.

The sequence, chemical modifications and structure of oligonucleotides used in Example 1 are shown in Table 1 and Figure 3. All the oligonucleotides were manufactured by GeneDesign Inc. (Osaka, Japan).

[Table 1]

| Name of nucleic acid agent | Name of oligonucleotide | Sequence (5' -3') | SEQ ID NO: |
|---|---|---|---|
| PMO | PMO | **g•g•c•c•a•a•a•c•c•t•c•g•g•c•t•t•a•c•c•t•g•a•a•a•t** | 1 |
| Toc-HDO (default) | First nucleic acid strand: PMO | **g•g•c•c•a•a•a•c•c•t•c•g•g•c•t•t•a•c•c•t•g•a•a•a•t** | 1 |
| | Second nucleic acid strand: Toc-cRNA | Toc-A*U*U*UCAGGUAAGCCGAGGUUUG*G*C*C | 2 |
| Chol-HDO (default) | First nucleic acid strand: PMO | **g•g•c•c•a•a•a•c•c•t•c•g•g•c•t•t•a•c•c•t•g•a•a•a•t** | 1 |
| | Second nucleic acid strand: Chol -cRNA | Chol-A*U*U*UCAGGUAAGCCGAGGUUUG*G*C*C | 2 |
| Chol -HDO (CU -OMe) | First nucleic acid strand: PMO | **g•g•c•c•a•a•a•c•c•t•c•g•g•c•t•t•a•c•c•t•g•a•a•a•t** | 1 |
| | Second nucleic acid strand: Chol -cRNA (CU -OMe) | Chol-A*U*U*UCAGGUAAGCCGAGGUUUG*G*C*C | 3 |

(continued)

| Name of nucleic acid agent | Name of oligonucleotide | Sequence (5' -3') | SEQ ID NO: |
|---|---|---|---|
| Chol -HDO (CT-DNA) | First nucleic acid strand: PMO | **g•g•c•c•a•a•a•c•c•t•c•g•g•c•t•t•a•c•c•t•g•a•a•a•t** | 1 |
| | Second nucleic acid strand: Chol -cRNA (CT-DNA) | Chol-A*U*U*tcAGGtAAGccGAGGtttG*G*C*C | 4 |
| Lower case letter (bold letter): morpholino nucleic acid •: Phosphorodiamidate bond<br>Upper case letter (underlined): 2' -O-Me RNA *: Phosphorothioate bond<br>Upper case letter: RNA Lower case letter (underlined): DNA<br>Toe: Tocopherol Chol: Cholesterol | | | |

(2) *In vivo* experiment

Mice were 6- to 7-week-old male mdx mice (Clair, Japan) weighing 20 g. Experiments using mice were performed with n = 2 to 4 (mainly n = 4). Nucleic acid agents, namely Chol-HDO (CU-OMe) and Chol-HDO (CT-DNA), were injected intravenously into the mice at a dose of 10 mg/kg each, once a week for a total of five times. Furthermore, as a negative control group, mice injected with PBS only, a single-stranded nucleic acid agent consisting of PMO, Toc-HDO (default), and Chol-HDO (default), respectively, were prepared.

(3) RNA expression analysis

Two weeks after the final administration of the nucleic acid agent, the mice were dissected to isolate heart muscle, diaphragm, paraspinal muscle, quadriceps, tibialis anterior, and triceps. Subsequently, mRNA was extracted from each tissue with Isogen II (manufactured by Nippon Gene Co., Ltd.). One-Step RT-PCR was performed on 500 ng of extracted total RNA using the Qiagen One Step RT-PCR Kit (Qiagen). A reaction solution was prepared according to the protocol provided with the kit. LifeECO (Manufactured by Bioer Technology Co. Ltd.) was used as the thermal cycler. The RT-PCR program used was as follows.

42°C for 30 min: reverse transcription reaction
95°C for 15 min: heat denaturation
[94°C for 30 sec, 60°C for 30 sec, 72°C for 60 sec] x 35 cycles: PCR amplification
72°C for 7 min: final elongation reaction

The base sequences of the forward primer and the reverse primer used for RT-PCR are as follows.

Forward primer: 5'-ATCCAGCAGTCAGAAAGCAAA-3' (SEQ ID NO: 5)
Reverse primer: 5'-CAGCCATCCATTTCTGTAAGG-3' (SEQ ID NO: 6)

One microliter of the reaction product of the above-described RT-PCR was analyzed using the Agilent DNA1000 kit with a Bioanalyzer 2100 (manufactured by Agilent Inc.). The amount (mol number) of polynucleotide "A" in the band where exon 23 was skipped and the amount (mol number) of polynucleotide "B" in the band where exon 23 was not skipped were measured. Based on these "A" and "B" measurements, the skipping efficiency was calculated according to the following formula.

$$\text{Skipping efficiency (\%)} = A/(A + B) \times 100$$

(Results)

The results are shown in Figures 4 to 9. In the heart muscle, Chol-HDO (default) showed skipping efficiency of 1.4%, whereas Chol-HDO (CU-OMe) and Chol-HDO (CT-DNA) showed skipping efficiency of 7.4% to 10.4%, representing about 5 to 7-fold increases of skipping efficiency (Figure 4). In the diaphragm, Chol-HDO (default) showed skipping efficiency of approximately 3%, whereas Cho-HDO (CU-OMe) and Cho-HDO (CT-DNA) showed skipping efficiency of 27% to 28%, representing approximately 9 to 10-fold increases of skipping efficiency (Figure 5). In the skeletal

muscle (paraspinal muscle, quadriceps, tibialis anterior, and triceps), Chol-HDO (default) showed skipping efficiency of approximately 3%, whereas Cho-HDO (CU-OMe) and Cho-HDO (CT-DNA) showed skipping efficiency of 27% to 30%, representing 9 to 10-fold increases of skipping efficiency (Figures 6 to 9). The above-described results have revealed that Cho-HDO (CU-OMe) and Cho-HDO (CT-DNA) dramatically increased the efficiency of exon skipping compared with Chol-HDO (default) and PMO.

<Example 2: Evaluation of stability of heteronucleic acid PMO in human spinal fluid>

(Purpose)

The stability of a double-stranded nucleic acid complex comprising a morpholino nucleic acid (the first nucleic acid strand) and cRNA or cDNA (the second nucleic acid strand) as a complementary strand thereof was evaluated in the human spinal fluid.

(Method)

(1) Preparation of nucleic acids

HDO (PMO/cRNA) and HDO (PMO/cDNA) described in Table 2 below were produced by the same method as in Example 1. HDO (PMO/cRNA) is a double-stranded nucleic acid complex comprising morpholino nucleic acids (the first nucleic acid strand) and a complementary strand (the second nucleic acid strand) thereof which consists of RNA. The second nucleic acid strand of HDO (PMO/cRNA) was not bound to a ligand. HDO (PMO/cDNA) is a double-stranded nucleic acid complex comprising morpholino nucleic acids (the first nucleic acid strand) and a complementary strand (the second nucleic acid strand) thereof which consists of DNA. The second nucleic acid strand of HDO (PMO/cDNA) was not bound to a ligand.

[Table 2]

| Name of nucleic acid agent | Name of oligonucleotide | Sequence (5' -3') | SEQ ID NO: |
|---|---|---|---|
| HDO (PMO/cRNA) | First nucleic acid strand: PMO | **g•g•c•c•a•a•a•c•c•t•c•g•g•c•t•t•a•c•c•t•g•a•a• a•t** | 1 |
| | Second nucleic acid strand: cRNA | AUUUCAGGUAAGCCGAGGUUUGGCC | 17 |
| HDO (PMO/cDNA) | First nucleic acid strand: PMO | **g•g•c•c•a•a•a•c•c•t•c•g•g•c•t•t•a•c•c•t•g•a•a• a•t** | 1 |
| | Second nucleic acid strand: cDNA | <u>atttcaggtaagccgaggtttggcc</u> | 18 |
| Lowercase letter (bold letter): morpholino nucleic acid •: Phosphorodiamidate bond<br>Upper case letter: RNA Lower case letter (underlined): DNA | | | |

(2) Evaluation of stability in human spinal fluid

Each of HDO (PMO/cRNA) and HDO (PMO/cDNA) was prepared at 20 μM in an amount of 1.2 μL. With 1.2 μL of 20 μM nucleic acids, 4.8 μL of human spinal fluid was mixed, and the resulting mixture was incubated in an incubator at 37°C. At each time point after 0 minutes, 10 minutes, 6 hours, 24 hours, and 5 days, the mixture was introduced into liquid nitrogen to terminate the reaction.

(3) Electrophoresis and analysis

Acrylamide gel (1× TBE) at 16% was prepared and produced. The electrophoresis sample was electrophoresed in the gel at 100 V for 80 minutes. As controls, a PMO alone, a cRNA alone, and a cDNA alone were simultaneously electrophoresed. Then, a solution was produced by diluting, with 1× TBE, an aqueous GelRed (× 10000) solution (Biotium) to a 1/10000 concentration. The gel was permeated with the solution for 10 minutes. Then, the gel was

photographed with a ChemiDoc Touch imaging system (Bio-Rad Laboratories, Inc.).

(Results)

The electrophoresis results of HDO (PMO/cRNA) and HDO (PMO/cDNA) before and after being mixed and incubated with human spinal fluid are shown in Figure 10. In the case of HDO (PMO/cRNA), band shifts in the direction of lower mobility was observed as the sample was incubated in the human spinal fluid (left side in Figure 10; after 6 hours to 5 days; in the frame indicated by the dotted line). The band shifts showed that, in the case of HDO (PMO/cRNA), the complementary strand was degraded in the human spinal fluid, thus generating a single-stranded PMO molecule. On the other hand, in the case of HDO (PMO/cDNA), no such band shifts were observed, indicating that the double-stranded form was stable (right side in Figure 10). The above-described results have revealed that use of DNA as a complementary strand dramatically increased the stability of the double-stranded nucleic acid complex in the human spinal fluid.

<Example 3: Evaluation of stability of ligand-bound heteronucleic acid PMO in human spinal fluid>

(Purpose)

The stability of the nucleic acid agents produced in Example 1 in the human spinal fluid is evaluated.

(1) Preparation of nucleic acids

Chol-HDO (default), Chol-HDO (CU-OMe), and Chol-HDO (CT-DNA) described in Table 1 were produced by the same method as in Example 1.

(2) Evaluation of stability in human spinal fluid

Each of Chol-HDO (default), Chol-HDO (CU-OMe), and Chol-HDO (CT-DNA) was prepared at 20 μM in an amount of 1.2 μL. For 1.2 μL of 20 μM nucleic acids, 4.8 μL of human spinal fluid was mixed, and the resulting mixture was incubated in an incubator at 37°C. At each time point at 0 minute and after 6 hours and 24 hours, the mixture was introduced into liquid nitrogen to terminate the reaction.

(3) Electrophoresis and analysis

Acrylamide gel at 16% (1× TBE) was prepared and produced. The electrophoresis sample described above was electrophoresed in the gel at 100V for 80 minutes. Then, a solution was produced by diluting, with 1× TBE, an aqueous GelRed (× 10000) solution (Biotium) to a 1/10000 concentration. The gel was permeated with the solution for 10 minutes. Then, the gel was photographed with a ChemiDoc Touch imaging system (Bio-Rad Laboratories, Inc.).

(Results)

The electrophoresis results of Chol-HDO (default), Chol-HDO (CU-OMe), and Chol-HDO (CT-DNA) were shown in Figures 11A to 11C. In each panel, the three lanes represent the time points after 0 minutes, 6 hours, and 24 hours after the nucleic acid and the spinal fluid were mixed.

In Figure 11A, two bands were observed (the first lane from the left) when Chol-HDO (default) and the spinal fluid were mixed, but decreases in the intensity of both bands were observed after 6 hours (the second lane from the left). After 24 hours, further decreased in the intensity were observed (the third lane from the left).

On the other hand, according to Figure 11B and Figure 11C, a single band was observed when Chol-HDO (CU-OMe) and Chol-HDO (CT-DNA) were mixed with the spinal fluid, and the intensity did not change even after 6 hours or 24 hours. Accordingly, it was demonstrated that Chol-HDO (CU-OMe) and Chol-HDO (CT-DNA) have high stability in the spinal fluid.

All publications, patents, and patent applications cited herein are incorporated herein directly by reference.

Sequence Listing

## Claims

1. A double-stranded nucleic acid complex comprising a first nucleic acid strand and a second nucleic acid strand, wherein:

    said first nucleic acid strand comprises an artificial nucleic acid, and is capable of hybridizing to at least part of a target gene or a transcription product thereof, and can induce exon skipping, exon inclusion and/or steric blocking to said target gene or transcription product thereof,
    said second nucleic acid strand comprises a base sequence complementary to at least part of said first nucleic acid strand, and comprises at least one blocking region which is resistant to a nuclease which is present in a body fluid, and at least one cleavage region which undergoes degradation by a nuclease which is present intracellularly, and
    said cleavage region comprises two or more consecutive sugar-unmodified nucleosides.

2. The double-stranded nucleic acid complex of claim 1, wherein said artificial nucleic acid comprises any one or more of a morpholino nucleic acid, a 2'-O-methyl-modified nucleoside, a 2'-O-methoxyethyl-modified nucleoside, a tricyclo-DNA, a peptide nucleic acid, or a 2'-O-[2-(N-methylcarbamoyl)ethyl]-modified nucleoside.

3. The double-stranded nucleic acid complex of claim 1 or 2, wherein said blocking region comprises at least one or more selected from the group consisting of a deoxyribonucleoside, a 2'-modified nucleoside, a 5'-modified nucleoside, and a bridged nucleoside which comprises a pyrimidine base.

4. The double-stranded nucleic acid complex of claim 3, wherein said 2'-modified nucleoside is selected from the group consisting of a 2'-O-methyl-modified nucleoside, a 2'-O-methoxyethyl-modified nucleoside, and a 2'-O-[2-(N-methylcarbamoyl)ethyl]-modified nucleoside.

5. The double-stranded nucleic acid complex of claim 3 or 4, wherein said 5'-modified nucleoside is a 5'-cp-modified nucleoside, a 5'-methyl-modified nucleoside, or a 5'-dimethylmodified nucleoside.

6. The double-stranded nucleic acid complex of any one of claims 3 to 5, wherein said bridged nucleoside is selected from the group consisting of an LNA nucleoside, a 2',4'-BNA$^{NC}$ nucleoside, a cEt BNA nucleoside, an ENA nucleoside, an AmNA nucleoside, a GuNA nucleoside, an scpBNA nucleoside, an scpBNA2 nucleoside, and a BANA3 nucleoside.

7. The double-stranded nucleic acid complex of any one of claims 1 to 6, wherein said second nucleic acid strand does not comprise two or more consecutive natural ribonucleosides comprising a pyrimidine base.

8. The double-stranded nucleic acid complex of any one of claims 1 to 7, wherein all of the nucleosides that comprise a pyrimidine base in said second nucleic acid strand are deoxyribonucleosides, 2'-modified nucleosides, 5'-modified nucleosides, bridged nucleosides, or a combination thereof.

9. The double-stranded nucleic acid complex of any one of claims 1 to 8, wherein said cleavage region comprises one to ten consecutive sugar-unmodified nucleosides.

10. The double-stranded nucleic acid complex of any one of claims 1 to 9, wherein the nucleic acids in said first nucleic acid strand consist of morpholino nucleic acids.

11. The double-stranded nucleic acid complex of any one of claims 1 to 10, wherein said second nucleic acid strand comprises a non-complementary base, and/or one or more insertion sequences and/or deletions, relative to said first nucleic acid strand.

12. The double-stranded nucleic acid complex of claim 11, wherein said second nucleic acid strand comprises one to three said non-complementary bases.

13. The double-stranded nucleic acid complex of claim 11 or 12, wherein said insertion sequence consists of one to eight bases.

14. The double-stranded nucleic acid complex of any one of claims 11 to 13, wherein said deletion consists of consecutive

one to four bases.

**15.** The double-stranded nucleic acid complex of any one of claims 1 to 14, wherein: in said sugar-unmodified nucleosides, the internucleoside linkage between a deoxyribonucleoside and a deoxyribonucleoside is a phosphodiester bond, and the internucleoside linkage between a deoxyribonucleoside and a ribonucleoside or the internucleoside linkage between a ribonucleoside and a ribonucleoside is a phosphodiester linkage and/or a phosphorothioate bond.

**16.** The double-stranded nucleic acid complex of any one of claims 1 to 15, wherein said second nucleic acid strand is at least 8 bases in length.

**17.** The double-stranded nucleic acid complex of any one of claims 1 to 16, wherein the base length of said second nucleic acid strand is shorter than the base length of the first nucleic acid strand.

**18.** The double-stranded nucleic acid complex of any one of claims 1 to 17, wherein said second nucleic acid strand comprises at least one overhang region at one or both of the 5' end side and 3' end side thereof.

**19.** The double-stranded nucleic acid complex of claim 18, wherein said overhang region is one to 20 bases in length.

**20.** The double-stranded nucleic acid complex of claim 18 or 19, wherein said overhang region comprises at least one deoxyribonucleoside and/or non-natural nucleoside.

**21.** The double-stranded nucleic acid complex of any one of claims 1 to 20, wherein said second nucleic acid strand comprises at least one non-natural nucleoside at the 5' end and/or 3' end thereof.

**22.** The double-stranded nucleic acid complex of any one of claims 1 to 21, wherein said second nucleic acid strand is bound to a ligand.

**23.** The double-stranded nucleic acid complex of claim 22, wherein said ligand is any one or more selected from the group consisting of a small molecule, a peptide, a lipid, and a nucleic acid aptamer.

**24.** The double-stranded nucleic acid complex of claim 23, wherein said peptide is an antibody or an active fragment thereof.

**25.** The double-stranded nucleic acid complex of claim 23, wherein said lipid is cholesterol or an analog thereof, or tocopherol or an analog thereof.

**26.** The double-stranded nucleic acid complex of any one of claims 22 to 25, wherein said ligand is bound to the 5' end and/or 3' end of said second nucleic acid strand.

**27.** The double-stranded nucleic acid complex of any one of claims 1 to 26, wherein all or a part of the internucleoside linkages in said first nucleic acid strand and/or said second nucleic acid strand is a modified internucleoside linkage.

**28.** The double-stranded nucleic acid complex of claim 27, wherein said modified internucleoside linkage is a phosphorothioate linkage and/or a boranophosphate linkage.

**29.** The double-stranded nucleic acid complex of any one of claims 1 to 28, wherein said first nucleic acid strand and said second nucleic acid strand are bound via a linker.

**30.** The double-stranded nucleic acid complex of claim 29, wherein said linker consists of nucleic acids.

**31.** The double-stranded nucleic acid complex of any one of claims 1 to 30, wherein said body fluid is blood, spinal fluid, or lymph fluid.

**32.** A pharmaceutical composition comprising the double-stranded nucleic acid complex of any one of claims 1 to 31 as an active ingredient.

**33.** The pharmaceutical composition of claim 32 for increasing RNA expression or reducing RNA expression by exon skipping, exon inclusion and/or steric blocking, or regulating splice variants.

**34.** The pharmaceutical composition of claim 32 or 33 for treating skeletal muscle dysfunction or cardiac dysfunction.

**35.** The pharmaceutical composition of claim 34, wherein said skeletal muscle dysfunction or cardiac dysfunction is muscular dystrophy.

**36.** The pharmaceutical composition of claim 35, wherein said muscular dystrophy is myotonic dystrophy or Duchenne muscular dystrophy.

**37.** The pharmaceutical composition of claim 32 or 33 for treating brain disorder, spinal cord disorder, or peripheral nerve disorder.

**38.** The pharmaceutical composition of any one of claims 32 to 37, wherein the pharmaceutical composition is administered by intravenous administration, intramuscular administration, intraocular administration, transnasal administration, oral administration, inhalation administration, or subcutaneous administration.

**39.** The pharmaceutical composition of claim 38, wherein 0.1 mg/kg to 100 mg/kg of said double-stranded nucleic acid complex is administered.

**40.** The pharmaceutical composition of any one of claims 32 to 37, wherein the pharmaceutical composition is administered by intraventricular administration or intrathecal administration.

**41.** The pharmaceutical composition of claim 40, wherein said intrathecal administration is lumbar puncture.

**42.** The pharmaceutical composition of claim 40 or 41, wherein said double-stranded nucleic acid complex is administered at 0.1 mg to 200 mg.

**43.** The pharmaceutical composition of any one of claims 32 to 42, wherein the stability of said double-stranded nucleic acid complex in the blood and/or spinal fluid is improved.

# Fig. 1

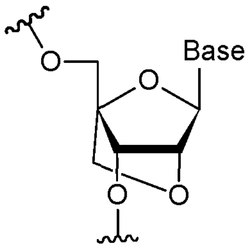

LNA

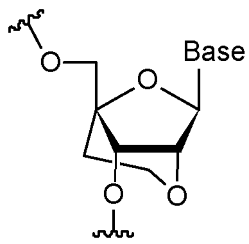

ENA

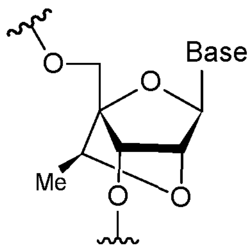

cEt

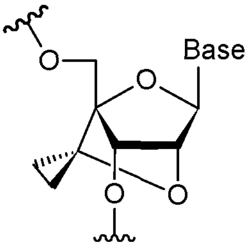

2'-O,4'-C-spirocyclopropylene bridged nucleic acid (scpBNA)

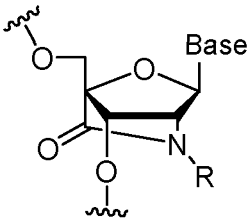

Amide bridged nucleic acid (AmNA) (R=H: AmNA[N-H], R=Me: AmNA[N-Me])

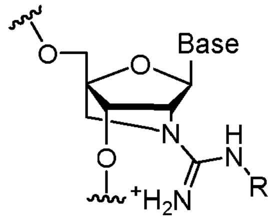

Guanidine bridged nucleic acid (GuNA) (R=H: GuNA[N-H], R=Me: GuNA[N-Me])

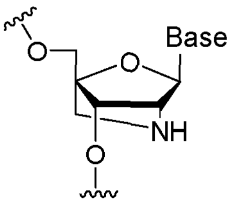

Amine bridged nucleic acid (2′- Amino-LNA)

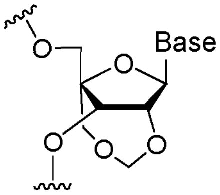

2',4'- BNA$^{COC}$

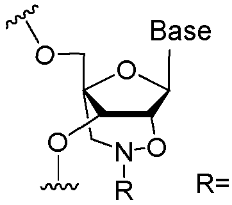

R=Me, H

2',4'- BNA$^{NC}$ (R=H: 2',4'-BNA$^{NC}$[N-H], R=Me: 2',4'-BNA$^{NC}$[N-Me])

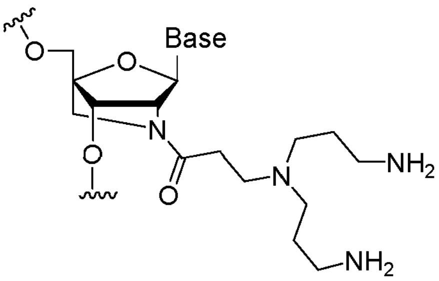

Amine bridged nucleic acid (2′- Amino-LNA) (3-(Bis(3-aminopropyl)amino)propanoyl substitution)

Fig. 2

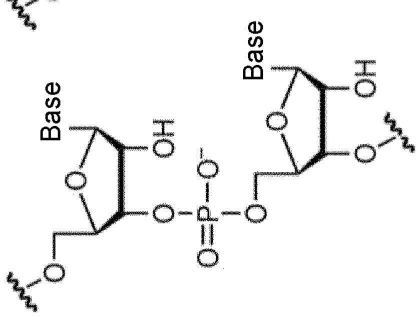
Phosphodiester

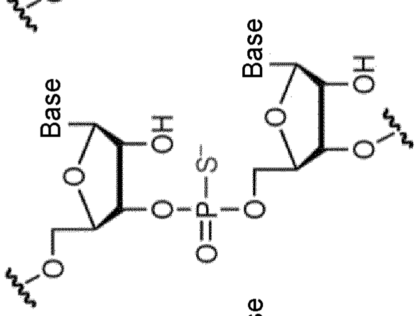
Phosphorothioate

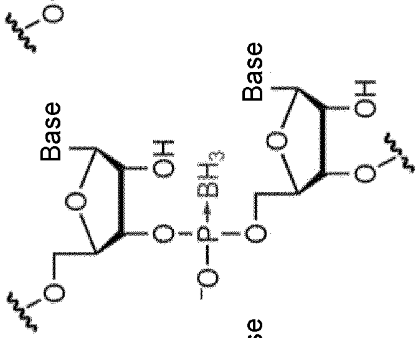
Boranophosphate

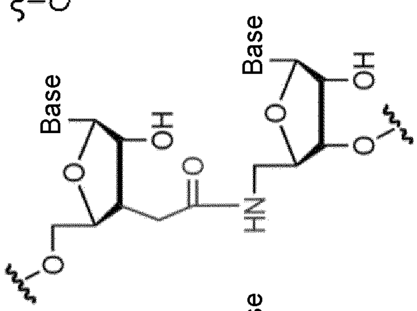
Amide bond

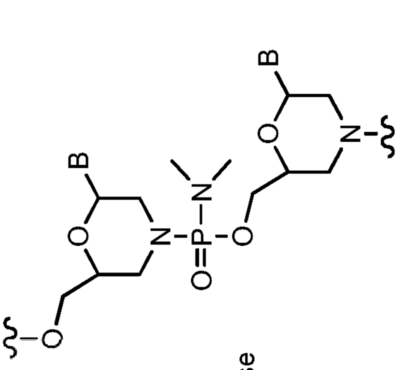
Morpholino

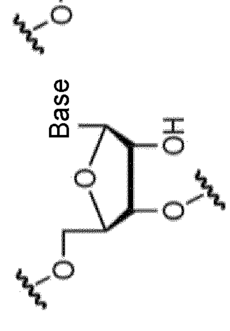
**RNA**

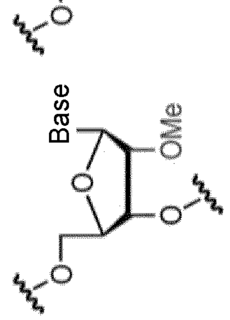
**2'-O-Me-RNA**

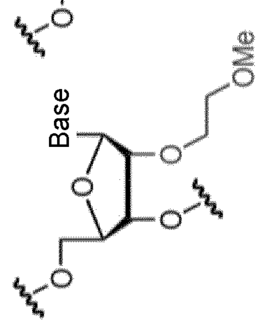
**2'-O-MOE-RNA**

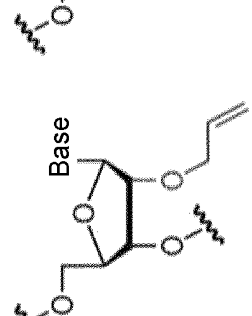
2'-O-allyl -RNA

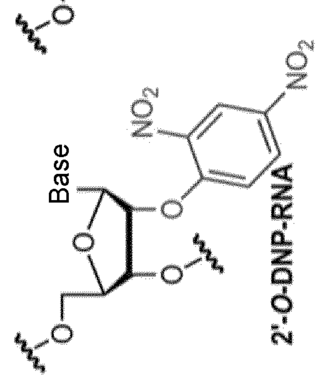
**2'-O-DNP-RNA**

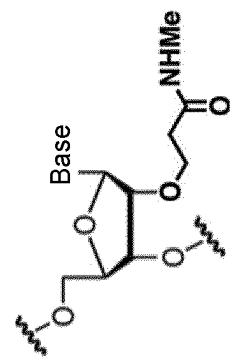
2'-O-MCE-RNA

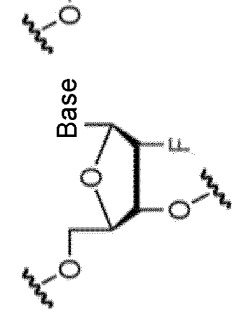
**2'F-RNA**

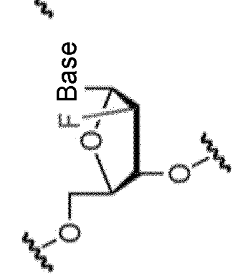
**2'F-ANA**

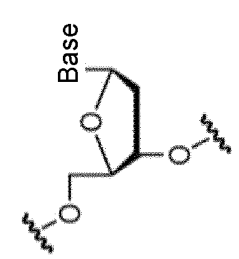
**DNA**

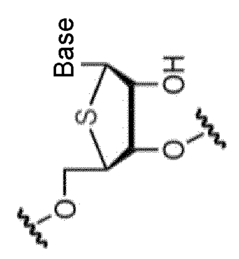
**4'S-RNA**

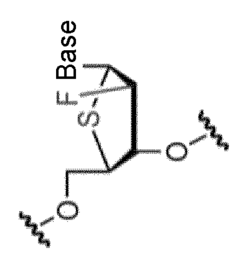
**4'S-FANA**

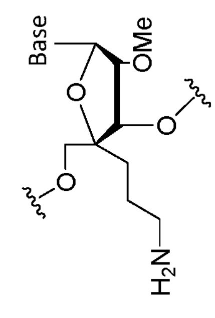
Aminoalkyl modified nucleic acid (2'-O-methyl and 4'-$CH_2CH_2CH_2NH_2$ substitution)

# Fig. 3

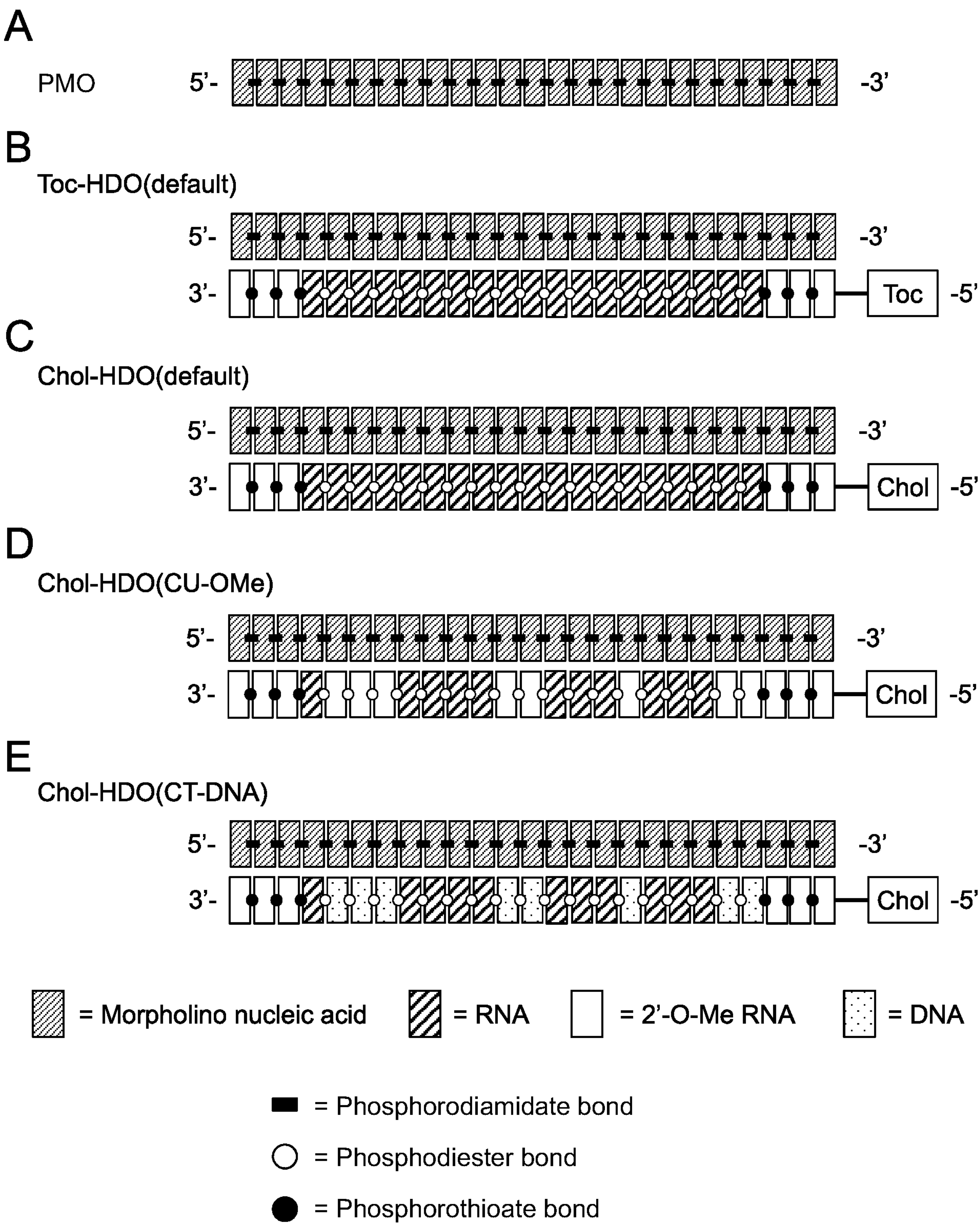
A
PMO
5'-
-3'
B
Toc-HDO(default)
5'-
-3'
3'-
Toc
-5'
C
Chol-HDO(default)
5'-
-3'
3'-
Chol
-5'
D
Chol-HDO(CU-OMe)
5'-
-3'
3'-
Chol
-5'
E
Chol-HDO(CT-DNA)
5'-
-3'
3'-
Chol
-5'
= Morpholino nucleic acid
= RNA
= 2'-O-Me RNA
= DNA
= Phosphorodiamidate bond
= Phosphodiester bond
= Phosphorothioate bond

Fig. 4

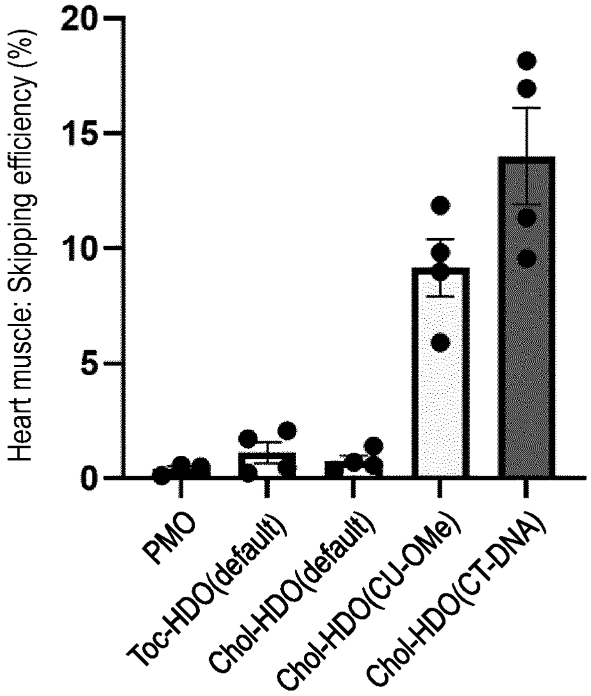
20
15
10
5
0
Heart muscle: Skipping efficiency (%)
PMO
Toc-HDO(default)
Chol-HDO(default)
Chol-HDO(CU-OMe)
Chol-HDO(CT-DNA)

Fig. 5

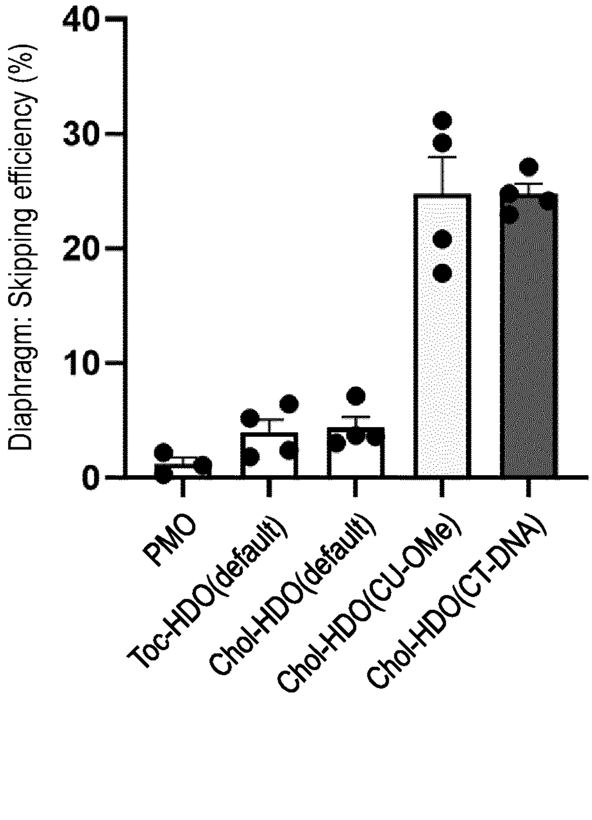
Diaphragm: Skipping efficiency (%)
40
30
20
10
0
PMO
Toc-HDO(default)
Chol-HDO(default)
Chol-HDO(CU-OMe)
Chol-HDO(CT-DNA)

Fig. 6

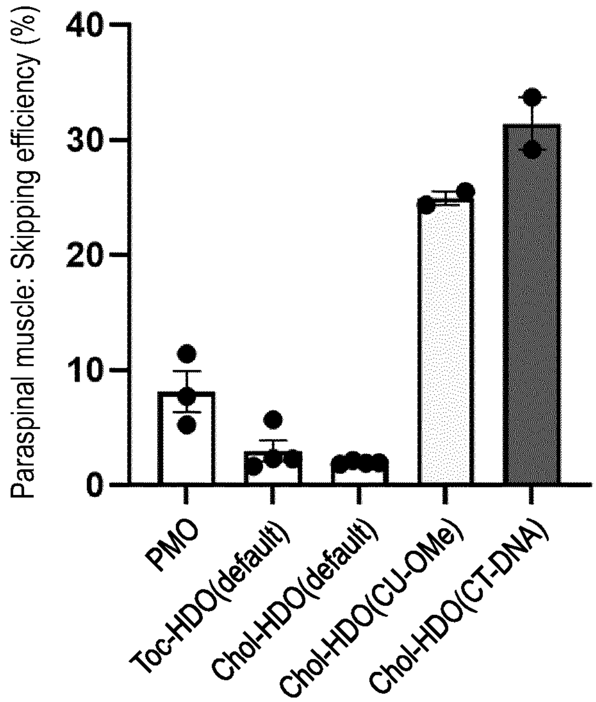
Paraspinal muscle: Skipping efficiency (%)
40
30
20
10
0
PMO
Toc-HDO(default)
Chol-HDO(default)
Chol-HDO(CU-OMe)
Chol-HDO(CT-DNA)

Fig. 7

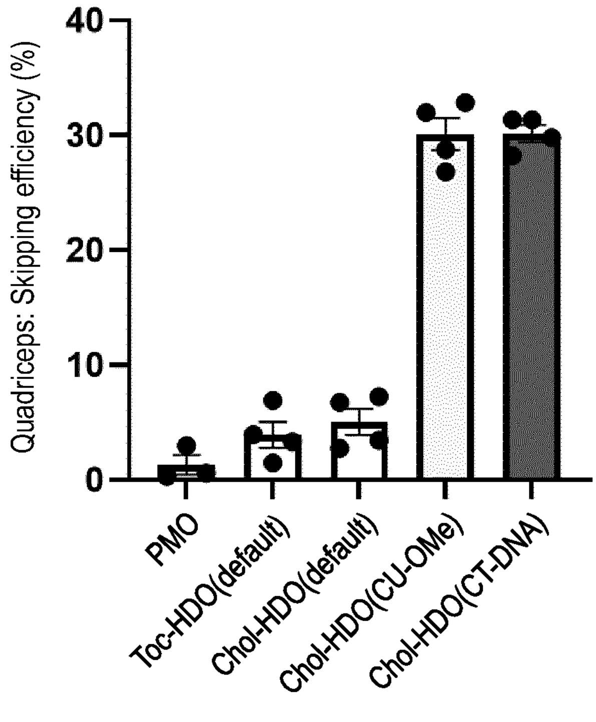
Quadriceps: Skipping efficiency (%)
40
30
20
10
0
PMO
Toc-HDO(default)
Chol-HDO(default)
Chol-HDO(CU-OMe)
Chol-HDO(CT-DNA)

Fig. 8

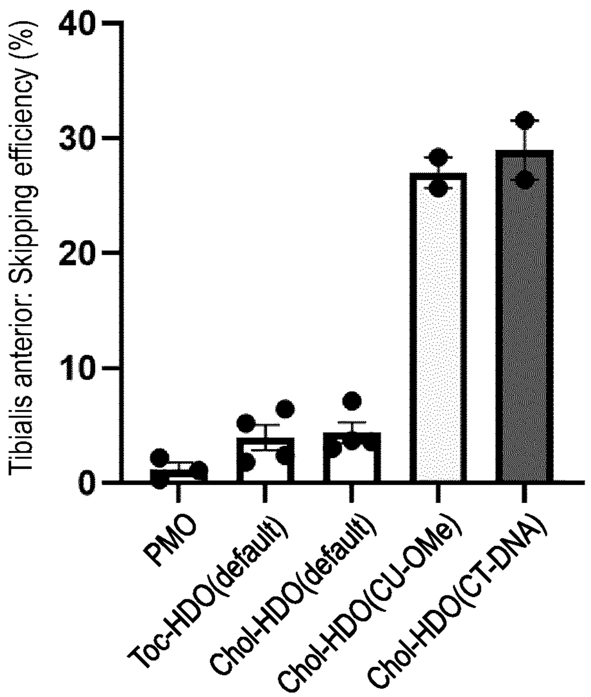
Tibialis anterior: Skipping efficiency (%)
40
30
20
10
0
PMO
Toc-HDO(default)
Chol-HDO(default)
Chol-HDO(CU-OMe)
Chol-HDO(CT-DNA)

Fig. 9

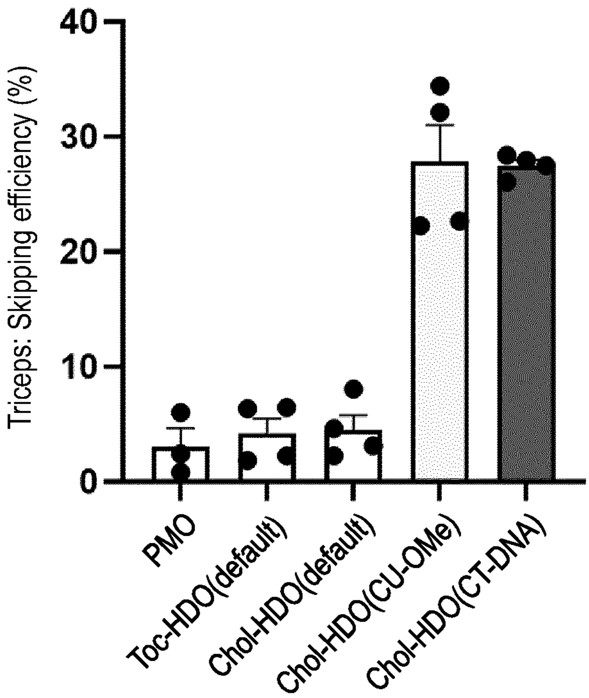
40
30
20
10
0
Triceps: Skipping efficiency (%)
PMO
Toc-HDO(default)
Chol-HDO(default)
Chol-HDO(CU-OMe)
Chol-HDO(CT-DNA)

Fig. 10

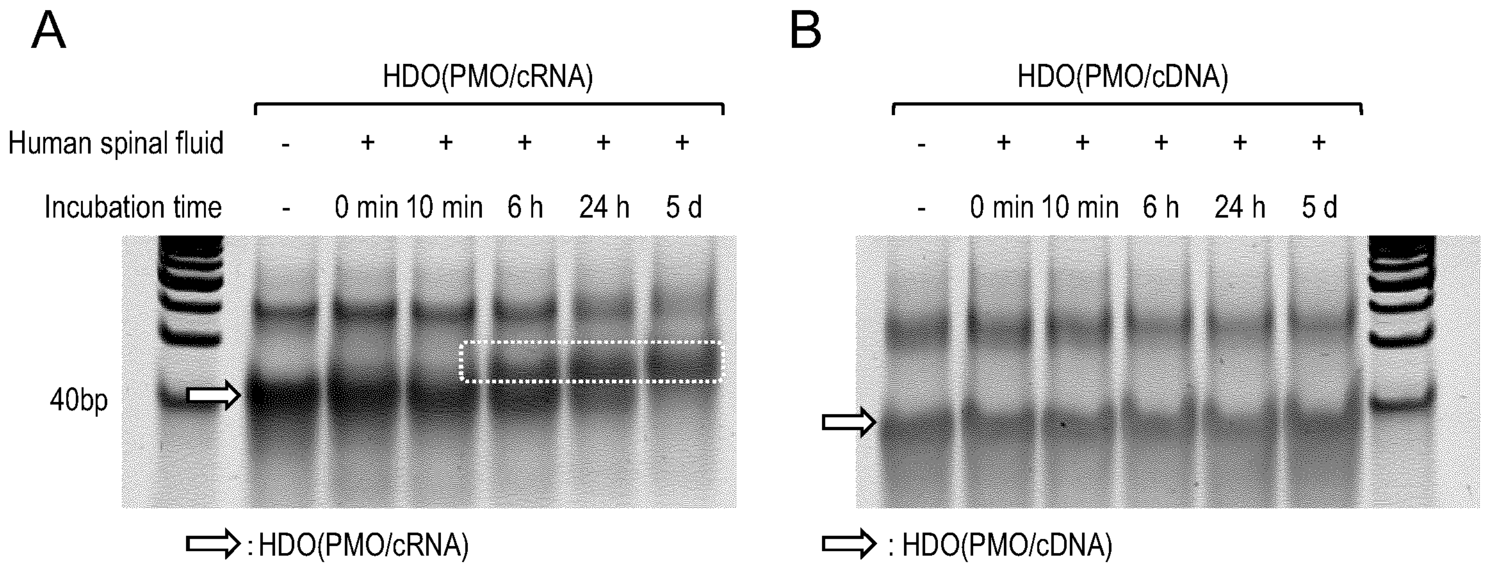
A
B
HDO(PMO/cRNA)
HDO(PMO/cDNA)
Human spinal fluid
- + + + + +
Incubation time
- 0 min 10 min 6 h 24 h 5 d
40bp
: HDO(PMO/cRNA)
: HDO(PMO/cDNA)

Fig. 11

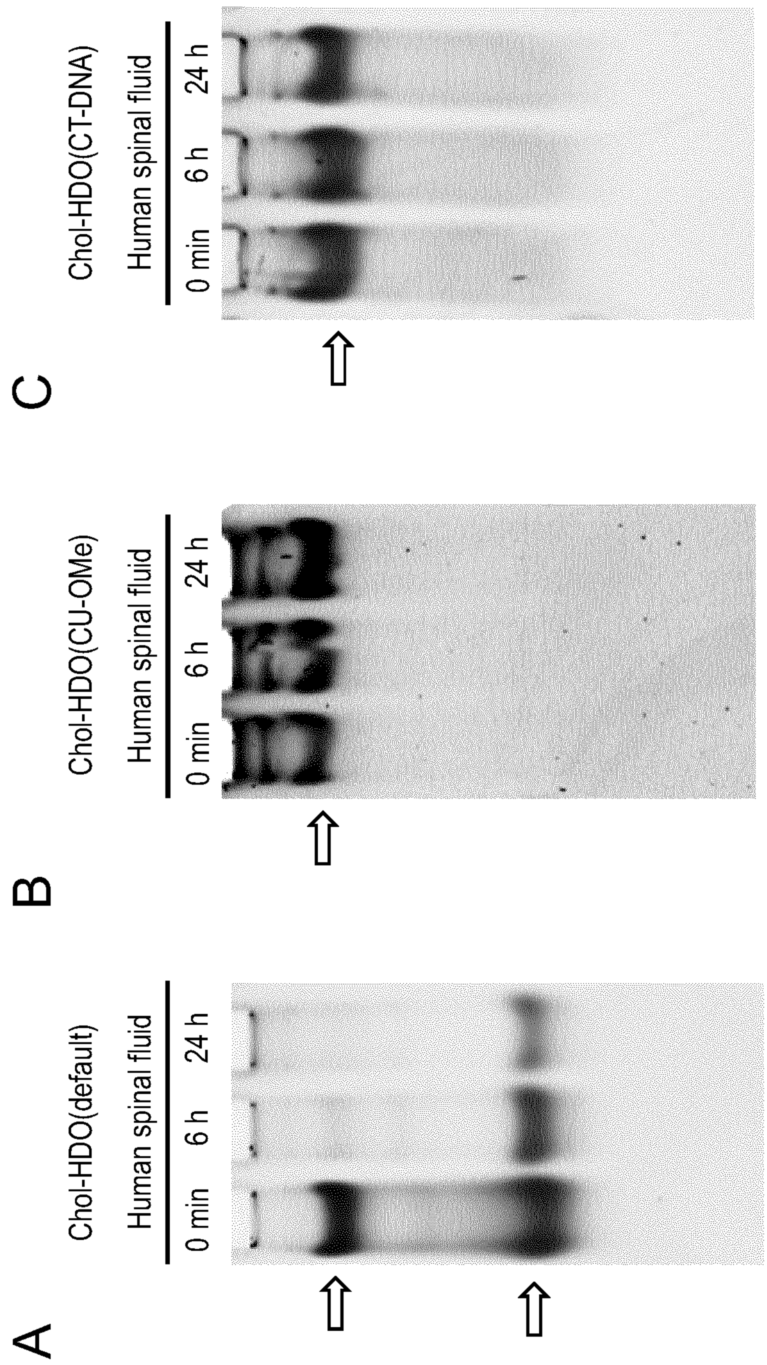
A
Chol-HDO(default)
Human spinal fluid
0 min
6 h
24 h
B
Chol-HDO(CU-OMe)
Human spinal fluid
0 min
6 h
24 h
C
Chol-HDO(CT-DNA)
Human spinal fluid
0 min
6 h
24 h

**INTERNATIONAL SEARCH REPORT**

International application No.
**PCT/JP2022/031385**

**A. CLASSIFICATION OF SUBJECT MATTER**

***C12N 15/113***(2010.01)i; ***A61K 31/713***(2006.01)i; ***A61K 48/00***(2006.01)i; ***A61P 21/00***(2006.01)i
FI: C12N15/113 Z; A61K31/713; A61K48/00; A61P21/00

According to International Patent Classification (IPC) or to both national classification and IPC

**B. FIELDS SEARCHED**

Minimum documentation searched (classification system followed by classification symbols)

C12N15/113; A61K31/713; A61K48/00; A61P21/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/209285 A1 (TOKYO MEDICAL AND DENTAL UNIVERSITY) 15 October 2020 (2020-10-15)<br>claims, paragraphs [0074], [0080], examples, drawings | 1-43 |

☐ Further documents are listed in the continuation of Box C.

☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 October 2022** | **01 November 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/031385**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "Annex C/ST.25 text file format" should be read as "ST.26 format".

Form PCT/ISA/210 (continuation of first sheet) (January 2015)

**INTERNATIONAL SEARCH REPORT**
**Information on patent family members**

International application No.
**PCT/JP2022/031385**

| Patent document cited in search report | Publication date (day/month/year) | Patent family member(s) | Publication date (day/month/year) |
|---|---|---|---|
| WO 2020/209285 A1 | 15 October 2020 | US 2022/0175817 A1<br>claims, paragraphs [0148], [0154], examples, drawings<br>EP 3954395 A1<br>CN 113677374 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013089283 A
- WO 2014192310 A
- JP 2021010214 W
- JP 2021134443 A
- WO 2007143315 A
- WO 2008043753 A
- WO 2008049085 A
- US 5955599 A
- US 5264423 A
- US 5286717 A
- WO 2015168172 A
- WO 2016081600 A
- JP 2019012077 W
- JP 2019010392 W
- JP 2020035117 W


### Non-patent literature cited in the description


- **NISHINA K.** DNA/RNA heteroduplex oligonucleotide for highly efficient gene silencing. *Nature Communication,* 2015, vol. 6, 7969
- **ASAMI Y et al.** Drug delivery system of therapeutic oligonucleotides. *Drug Discoveries & Therapeutics.,* 2016, vol. 10 (5), 256-262
- **ALEXANDER A. LOMZOV et al.** *Biochem Biophys Res Commun.,* 2019, vol. 513 (4), 807-811
- **NAOKI IWAMOTO et al.** *Angew. Chem. Int. Ed. Engl.,* 2009, vol. 48 (3), 496-9
- **NATSUHISA OKA et al.** *J. Am. Chem. Soc.,* 2003, vol. 125, 8307-8317
- **NATSUHISA OKA et al.** *J. Am. Chem. Soc.,* 2008, vol. 130, 16031-16037
- **YOHEI NUKAGA et al.** *J. Org. Chem.,* 2016, vol. 81, 2753-2762
- **YOHEI NUKAGA et al.** *J. Org. Chem.,* 2012, vol. 77, 7913-7922
- **NAOKI IWAMOTO et al.** *Nat. Biotechnol,* 2017, vol. 35 (9), 845-851
- **ANASTASIA KHVOROVA et al.** *Nat. Biotechnol.,* 2017, vol. 35 (3), 238-248